# EUROPEAN PATENT SPECIFICATION

(11) **EP 0 851 941 B1**
(45) Date of publication and mention of the grant of the patent: **17.11.1999**
(21) Application number: 96933834.2
(22) Date of filing: 19.09.1996
(51) Int. Cl.: C12Q 1/68

(54) **CONTROL FOR PCR**
EINE KONTROLLE FÜR DIE PCR
CONTROLES DE LA PCR

(30) Priority: 20.09.1995 US 4063 P
(43) Date of publication of application: 08.07.1998
(73) Proprietor: E.I. DU PONT DE NEMOURS AND COMPANY, Wilmington Delaware 19898 (US)
(72) Inventor: ANDALORO, Bridget, Walsh, Hockessin, DE 19707-1338 (US); BARBOUR, William, Mark, Newark, DE 19713-1201 (US); KING, Robert, Allen, Hockessin, DE 19707-9756 (US); KOPATSIS, Alexander, Demetrios, Wilmington, DE 19810-2733 (US); TICE, George, Jr., Penns Grove, NJ 08069-1952 (US); AMORESE, Douglas, Alan, Hockessin, DE 19707-9616 (US); JACKSON, Raymond, Edwin, Newark, DE 19713 (US); ECRET, Lisa, DiMarzio, Pennsville, NJ 08070 (US); ZANECOSKY, Heidi, Gertraud, Christiana, PA 17509 (US)
(74) Representative: Jones, Alan John
(86) International application number: US9615085
(87) International publication number: WO9711197

(56) References cited:
- EP-A- 0 379 369
- EP-A- 0 586 112
- WO-A-94/04706
- WO-A-94/16107
- US-A- 5 307 640
- MOLECULAR AND CELLULAR PROBES, vol. 5, no. 2, 1 April 1991, pages 151-156, XP000243966 YOLKEN R H ET AL: "SOLID PHASE CAPTURE METHOD FOR THE SPECIFIC AMPLIFICATION OF MICROBIAL NUCLEIC ACIDS-AVOIDANCE OF FALSE-POSITIVE AND FALSE-NEGATIVE REACTIONS"
- JOURNAL OF PATHOLOGY, vol. 162, 1 January 1990, pages 99-117, XP000386325 WRIGHT P A ET AL: "THE POLYMERASE CHAIN REACTION: MICRALE OR MIRAGE? A CRITICAL REVIEW OF ITS USES AND LIMITATIONS IN DIAGNOSIS AND RESEARCH"
- PATHOLOGY, vol. 26, 1994, pages 482-486, XP000644704 WEEKES ET AL.: "The diagnostic use of PCR for the detection of M. tuberculosis"

## Description

### FIELD OF THE INVENTION

This invention relates to the field of molecular biology and particularly to a rapid method for detection of selected microorganisms.

### BACKGROUND OF THE INVENTION

Detecting and indentifying bacteria is important in various medical and public health contexts and is important in controlling the quality of the food chain. Innumerable protocols, proprietary apparatus and kits have evolved to meet the needs of the rapidly growing field of bacterial detection. These require highly trained and skilled personnel to carry out the necessary procedures and even more highly trained and skilled personnel to evaluate the results. Moreover, many of the existing tests are extremely sensitive to environmental factors, such as growth and storage conditions and the presence of and competition from other bacteria or microorganisms. This puts an even greater emphasis on the need for exacting procedures and highly skilled operatives. Tests of the prior art are often expensive both with regard to reagents and to the apparatus in which the tests are run.

Additionally, these tests require confirmation since they often are not adequately selective or inclusive resulting in both false positive and false negative results.

Polymerase chain reaction (PCR) is a powerful analytical tool permitting the amplification of any desired specific nucleic acid sequence, contained in a nucleic acid or mixture thereof. The use of this procedure for bacterial detection has been reported in the literature. However, as it is commonly practiced, it is a procedure which puts demands adherance to strict protocols under strict conditions and requires personnel of advanced skills and training in order to achieve a reliable result.

In all DNA based methods for detection of organisms, and particularly in the PCR test procedure, extraneous components that may enhance or inhibit the test reaction make obtaining creditable results difficult. This occurs in testing food-derived matrices for bacterial contaminants that effect quality such as pathogens, spoilage, and off-taste promoters and the like. Because the test results in such circumstances are critical, it is important to evaluate the effectiveness of any particular test. The invention provides a positive control which is useful in establishing test validity.

The test procedure of the invention is PCR-based. In U.S. Patent 4,683,202, basic to that art, Mullis describes a procedure in which separate, complimentarystrands of the nucleic acid are treated with a molar excess of two oligonucleotide primers and the primers are extended to form complimentary primer extension products which act as templates for synthesizing the desired nucleic acid sequence. The steps of the reaction, a sequence of thermal treatments, are carried out stepwise or simultaneously and are repeated as often as desired. Typically as many as thirty-five or more cycles are necessary to obtain a number of replicas adequate for further processing.

In U.S. Patent 4,683,195, Mullis et al. teach that a specific nucleic acid sequence may be cloned into a vector by using primers to amplify the sequence which contain restriction sites on their non-complimentary ends and a nucleic acid fragment may be prepared from an existing shorter fragment using the amplification process.

PCR has several applications designed to detect the presence of a specific DNA sequence only by amplification. It would be extremely advantageous to include a control reaction in any such PCR test because, when a test is negative for a target, it is important to know if that result is true or if the reaction failed due to instrument malfunction or inhibition of the reaction due to sample matrix effects. The latter is particularly common in testing food samples to determine the presence of pathogens or other organisms harmful to product quality. Samples containing, for example, cocoa, a potent inhibitor of PCR, may well contain a pathogen that will be masked in an uncontrolled PCR-based test. A control reactant and method is the subject of the instant invention.

Wright et al., (J. Pathol., 162, 99, (1990)) teaches methods for performing polymerase chain reaction (PCR) and compares and contrasts the advantages and limitations of the method as a research and diagnostic tool. Specifically, Wright et al. discuss methods for eliminating false positives in PCR and notes that controls to eliminate false positives are contraindicated as they may lead to greater contamination of the reaction. Wright et al. suggest that separation of the control and the test reactions is one method of eliminating the contamination problem; however, they do not suggest or anticipate such a separation for the purpose of facilitating homogeneous detection of products.

Williams et al. in "DNA Polymorphisms Amplified by Arbitrary Primers are Useful as Genetic Markers", *Nucleic Acid Research,* Vol. 18, No. 22, p. 6531-6535 and Welsh et al., "Fingerprinting Genomes using PCR with Arbitrary Primers", *Nucleic Acid Research,* Vol. 18, No. 22, p. 7213-7218 both demonstrate the use of single, arbitrary primers in a DNA amplification reaction to generate a characteristic pattern of amplification products from genomic DNA from a variety of sources including bacteria. In WO93/11264, Jensen et al. teach the use of a single arbitrary primer across a broad spectrum of microorganisms. Control reactions are not addressed.

Shuldiner et al., in PB92-100932 NTIS, teach detecting an RNA sequence by tagging the sequence with a unique random nucleotide sequence during reverse transcription. The unique nucleotide sequence is then utilized to selectively amplify the resulting DNA sequence reducing the number of false positives obtained as a result of contaminating DNA such as from an endogenous source or from carry-over. This procedure lacks the control aspects of the instant invention which permit avoiding false negatives as well as false positives.

Tercero et al. (EP 586112), teach a vector useful as positive control in PCR amplification. The vector contains a sequence substantially identical to that of a primer used in the procedure which, after amplification, yields a product differing in size from that produced by the target. If only the vector is amplified the result is a true negative, but if neither vector nor target are amplified then the test must be faulty. Requisite in such a control protocol is some means to separate the different size products. Because the control and the target reactions are carried out in the same vessel and co-amplified, there are competing reactions that, in some circumstances reduce the sensitivity of the procedure. This results from preferential amplification of one of the targets. Also required are reference for size of product DNAs since in the case of only a single amplification product it must be determined whether it is test product or control product. Thus, the disclosure of Tercero et al. does not address homogeneous detection and is not adapted thereto.

In summary, the literature does not disclose a bacterial test method that 1) uses simplified molecular biology techniques that require no special skills in preparing and handling reagents and in carrying out the protocol, 2) is insensitive to environmental factors affecting phenotypic expression, and 3) is both selective and inclusive and has a positive control integrated into the protocol.

### SUMMARY OF THE INVENTION

Applicants have provided a method of reliable detection of the presence of a specific organism in a complex mixture. The steps of the method are
i) providing a tabletted control reagent for polymerase chain reactions (PCR) comprising aliquots of:
   a) an effective number of synthetic constructs of a characterizing DNA sequence structured with end sub-sequences that are complimentary to each other in reverse orientation;
   b) an effective number of units of a single primer complimentary to the 3' end of each strand of said construct;
   c) optionally an effective concentration of an intercalating dye;
   d) optionally, a stabilizer;
   e) an effective amount of a polymerase; and
   f) an effective amount of deoxy-nucleotide;
ii) providing a tabletted test reagent comprising an effective number of 761 primer and 35 primer and aliquots of c - f above; iii) providing a non-selectively enriched test solution comprising lysed bacteria from a natural matrix suspected of containing a target organism; iv) providing an effective solution of the solution of step iii with the tablet of step i in a single dose; v) providing an effective solution of the solution of step iii with the tablet of step ii in a single dose; vi) subjecting the solutions of steps iv and v to an effective number of PCR cycles; vii) adding a tracking dye to the resultant solutions of step vi; viii) subjecting the resultant solutions of step vii to electrophoretic separation (either PAGE or agarose gel) in parallel with a marker solution followed, optionally pre-stained with a nucleic acid-specific dye where said gel is not pre-stained with said nucleic acid-specific dye, by staining with a nucleic acid specific dye to produce identifying images; and ix) optically analyzing the resultant images of step viii to definitively identify the presence or absence of the target organism.

Preferably, the method effectively uses at least about 10 constructs. The final concentration of the single primer is from about 0.01 micromolar to about 0.5 micromolar. The optional intercalating dye is preferably selected from the group consisting of nucleic acid dyes and asymeytric cyanine dyes and the preferred effective concentration is from about 0.1 micromolar to about 6.0 micromolar. The preferred optional stabilizer is trehalose. The preferred synthetic sequence is modified recombinant pUC18 carrying *Salmonella* sequence with complimentary ends.

The control and test reagents used in the method are frozen into particles by means of a cryogenic liquid, said particles providing feedstock for tabletting.

Retrieval of the target bacterial sample from the non-selectively enriched test solution of Step iii) is accomplished by incubation in the presence of a piece of Porex™ high density polyethylene whereby removal and processing said piece effectively processes said target bacterial sample.

A further embodiment of the invention is a method of making a control reactant for a polymerase chain reaction (PCR), the steps of which are a) identifying a characterizing DNA sequence; b) synthesizing a construct of the characterizing sequence, said construct characterized by end sub-sequences that are complimentary to each other in reverse orientation terminating said sequence cloned from an amplified product of said characterizing sequence; c) providing an effective number of the constructs of step b) ; d) identifying a single priming DNA sequence that is unique to the construct and is complimentary to the priming sites of the end sub-sequences; e) providing an effective number of units of the single primer; f) optionally providing an effective amount of an intercalating dye; and g) optionally providing an effective amount of a stabilizer.

Preferably, the target bacterial sample was retrieved from the non-selectively enriched test solution of step iii) by a 1:10 dilution thereof followed by an effective grow-back incubation to provide an effective solution of the solution of step iii). Preferably, the control reagent is tabletted. Preferably, the "snow gun process" is used for producing frozen particles of the control reactant, the particles providing the feedstock for tabletting.

The invention further encompasses a method of testing for a target bacterium using a control reactant for a polymerase chain reaction (PCR), the steps including A) introducing into a control well a mixture of i) an effective amount of a control reactant comprising an effective number of copies of a single primer for a target DNA sequence, an effective number of copies of said target DNA sequence comprising a construct of an identifying sequence with end groups that are complimentary to each other in reverse orientation; ii) optionally an effective amount of a compatible intercalating dye that does not adversely affect PCR amplification; iii) an effective amount of a polymerase; iv) an effective amount of a nucleotide; and, v) optionally, an effective amount of a stabilizer; B) introducing into a test well a mixture of i) an effective amount of a test reactant comprising an effective amount of primers for a target sequence; ii) an effective amount of an intercalating dye that does not adversely affect PCR amplification; iii) an effective amount of a polymerase; iv) an effective amount of deoxy-nucleotides; and, v) optionally, an effective amount of a stabilizer; C) adding to both the test well and the control well in a single dose addition an analysate comprising a liquid carrier including a buffer compatible with said polymerase, said buffer dosed with a sample from a source possibly containing a target bacterium and organic and/or inorganic extraneous matter, said analysate optionally subjected to a lysing step before said additional step, and said lysate optionally prepared by a dilution and regrowth procedure; D) optionally mixing the contents in each of said wells; E) lysing the bacterial cells of the analysate by subjecting both wells to a lysing temperature in those instances where in step C) the lysing was not done before said introduction; F) heating the lysed contents of each well to effective pcr temperature and cycling for a selected number of cycles to provide a number of DNA copies sufficient for detection; G) following step F), causing the dye in each well to fluoresce; H) measuring light emitted from each of said wells; and I) recording the target bacterium as present when adequate measurable light emission is observed in both wells or recording the target bacterium as absent when there is no adequate measurable emission in said test well and there is adequate emission in said control well or recording a faulty test when there is no adequate emission from said test well; said adequacy of measurable light emission being determined by precalibration of light emission from the control reactant and comparison of the light emission from the control well and the test well after step H) with that calibration, deviations of light emission from the precalibrated value in the test well being ascribed to effects of extraneous matter as additive or subtractive, depending upon whether the observed value in said test well is above or below said calibrated value, permitting elimination of said effects.

Preferably, the control reactant and said test reactant are tabletted. Preferably, the intercalating dye has a DNA partitioning coefficient in a ten percent ethanol/water solution substantially equal to or lower than 1 x 10⁷.

The invention further encompasses a method of testing for a target bacterium using a control reactant, the steps including A) adding to a control well i) an effective amount of a control reactant comprising an effective amount of a single primer for a target DNA sequence; ii) an effective number of copies of said target DNA sequence comprising a construct of an identifying sequence with end groups that are on the opposite strand; iii) an effective amount of a polymerase; iv) an effective amount of a nucleatide; and, v) optionally, an effective amount of a stabilizer; B) adding to a test well i) an effective amount of a test reactant comprising an effective amount of primers specific to target DNA; ii) an effective amount of a polymerase; iii) an effective amount of a nucleotide; and, iv) optionally, an effective amount of a stabilizer; C) adding to both the test well and the control well an analysate comprising a liquid carrier including a buffer compatible with said polymerase, said buffer dosed with a sample of a suspect target bacterium derived from a source possibly containing organic and/or inorganic extraneous matter, said analysate optionally subjected to a lysing step before said introduction; D) optionally mixing the contents of each well; E) lysing the bacterial content of the test well by subjecting both wells to a lysing temperature in those instance where in step C) the optional lysing was not done before said introduction; F) heating the lysed contents of both wells to an effective pcr temperature and cycling for a selected number of cycles to provide a number of DNA replicates sufficient for detection; G) adding an effective amount of an intercalating dye to both wells; H) following step G), causing the dye in each well to fluoresce; I) measuring any light emitted from each of said wells; and J) identifying the target bacterium as present when adequate measurable emission is observed in both wells or identifying the target bacterium as absent when there is no adequate measurable emission in said test well and there is adequate emission in said control well or identifying the test as faulty when there is no adequate emission from said test well; said adequacy being determined by precalibration of emission from the control reactant and comparison of the emission from the control well and the test well after step H) with that calibration, deviations of emission from the precalibrated value in the test well being ascribed to effects of extraneous matter and additive or subtractive, depending upon whether the observed value in said test well is above or below said calibrated value, to eliminate said effects.

Preferably, the dosed sample of a suspect target bacterium is retrieved from a non-selective enrichment process by a 1:10 dilution thereof followed by an effective grow-back incubation to provide an effective analysate. Preferably, the test reactant and said control reactant are tabletted and the reactants are frozen by means of a cryogenic liquid into particles, the particles providing the feedstock for tabletting.

### BRIEF DESCRIPTION OF THE DRAWINGS

### BIOLOGICAL DEPOSIT, AND SEQUENCE LISTING

Figure 1 is a general flow diagram of the steps of the invention.

Figure 2 is a block diagram of the control construct, showing recombinant pUC containing 2 761 priming sites.

Figure 3 is a block diagram of the steps undertaken to make the construct of Figure 2.

Figure 4 is a gel electropheresis pattern of a step in preparing the construct.

Figure 5 is a gel electropheresis pattern showing a test result in which all clones were apparently the same size indicating no inserts.

Figures 6a, b and c are gel electropheresis patterns lanes that appeared to have inserts.

Figure 7 is a gel electrophoresis pattern indicating proper sized inserts in two clones with a Hind III site at 581 bp.

Figure 8 is a gel electropheresis pattern confirming the presence of the construct of the invention.

Figures 9a-c are a gel electropheresis pattern showing cloning of the 761 priming site into the previously constructed clone.

Figure 10 is a test protocol to demonstrate the utility of the construct plasmid as a control.

Figures 11a-d are a gel electropheresis pattern showing the result of the protocol of Figure 10 and confirming the utility of the control plasmid of the invention. This is internal refernce IV.

Figures 12a and b are is a gel electrophoresis pattern obtained in Example 1 illustrating the practice of the invention.

FigureS 13a-c show gel electrophoresis patterns and fluorescence measurements for Example 5 illustrating positive (a), negative (b), and invalid (c) results using the control of the invention in both polyacrylamide gel electrophoresis and homogeneous detection in a food-based system ordinarily difficult to control.

Figures 14a-f are PAGE based blots of tests run in Example 7.

Figure 15 shows the results of threshold testing and BAXä system validation for S. typhimurium.

Figure 16 shows the gel electrophoresis pattern obtained in Example 8 illustrating the practice of the invention.

By the filing date of this application, Applicants have made the following biological deposit under the terms of the Budapest Treaty on the International

Recognition of the Deposite of Micro-organisms for the Purposes of Patent Procedure:

| Depositor Identification Reference | Int'1. Depository Designation | Date of Deposit |
|---|---|---|
| p UC 18 (carrying *Salmonella* Sequence SEQ ID NO:1) | ATCC 97724 | September 19, 1996 |
| | | |

As used herein, "ATCC" refers to the American Type Culture Collection international depository located at 12301 Parklawn Drive, Rockville, MD 20852 U.S.A. The "ATCC No." is the accession number to cultures on deposit with the ATCC.

Applicants have provided one sequence listing in conformity with 37 C.F.R. 1.821-1.825 and Appendices A and B ("Requirements for Application Disclosures Containing Nucleotides and/or Amino Acid Sequences") and in conformity with "Rules for the Standard Representation of Nucleotide and Amino Acid Sequences in Patent Applications" and Annexes I and II to the Decision of the President of the EPO, published in Supplement No. 2 to OJ EPO, 12/1992.

### DETAILED DESCRIPTION OF THE INVENTION AND THE BEST MODE

The invention provides a control for PCR that is selective and inclusive, is integrated into the protocol, uses simplified molecular biology techniques that require no special skills in preparing and handling reagents and in carrying out the protocol and is insensitive to environmental factors affecting phenotypic expresion.

The method of the invention comprises three steps performed after conventional non-selective enrichment of an analysate sample suspected to contain a target organism. It is a DNA-based test and, as such, is insensitive to environmental factors affecting the phenotypic expression of the organism and, therefore, provides a high level of reliability and reproducibility.

The steps in sequence are DNA preparation, PCR amplification using primers specific to the target organism, and a positive control. The primers anneal to and amplify only target-specific sequences, and detection is done using a simple gel electrophoretic and staining procedure. The complexity of using PCR has been simplified through the use of pre-mixed lysing reagents and tabletted PCR reagents including both a test tablet and a control tablet which are packaged directly in the PCR tubes.

This invention also concerns a control reactant, its method of making and its method of use. By using the control reactant in parallel with a test reactant, assurance is provided that the processing cycle was operative. The control reactant, in tablet form, is a genetically-engineered (synthetic) construct of a characterizing DNA fragment, preferably from *Salmonella* with complimentary ends contained in a modified recombinant pUC 18 plasmid. It is used with an indicator, a single primer and, if tabletted as is preferred, a stabilizer.

Two general analytic procedures may be used.

The first is a simplified gel electrophoresis procedure. Instead of size-separating many DNA fragments, in the protocol of the invention only a single DNA fragment needs to be separated from the complex PCR reaction mixture. The sample is then examined for the presence or absence of the band formed by the fragment stained as by ethidium bromide staining. In addition to premixed and tabletted reagents, precast gels are used.

In use, according to the second general procedure, the analysate, is introduced to both a control well holding the single-primer control reactant and a parallel test well holding the standard two-primer test reactant. A suitable indicator, preferably a fluorescent dye, is added before or after entering the well. Similarly, the analysate is lysed before or after entering the well. Both wells are subjected to an appropriate number of PCR cycles. Detection follows. The contents are subjected to light of an appropriate wavelength and any fluorescence measured. A positive result in the test well is always considered a positive result regardless of the result in the control well. A positive result in the control well, regardless of the test well result, affirms the functioning of the process. Negative results in both wells must be considered inconclusive. Under some conditions the test well reaction may be calibrated and correction made for the effects of fluorescence enhancement or suppression by extraneous matter from the original sample, such as is common with some foodstuffs.

The invention is a method particular to a *Salmonella* screening product, improving the non-specific technology disclosed in W093/11264 to a method for the identification of microorganisms that closely meets the work flow and sampling needs of the food industry where this pathogen is an all-too-common contaminant that constitutes a serious health hazard to the consumers.

The method for tabletting used herein is termed "the Snow Gun process" and uses trehalose as a stabilizer. The technology is described in U.S. Patents 5,307,640 (Fawzey et al.); 4,762,857 (Bollin, Jr. et al.); 4,678,812 (Bollin, Jr. et al.), 3,932,943 (Briggs et al.), and U.S. Patent No. 5,475,984 (Application No. 08/298,231) (Fermani et al.). In general, the control and test reagents are frozen into particles by means of a cryogenic liquid, the particles providing feedstock for tabletting.

When used herein "PCR" means the Polymerase Chain Reaction as described by Mullis et al. in U.S. Patent 4,683,195 and Mullis in U.S. Patent 4,683,202.

"BAM" means the FDA Bacteriological Analytical Manual published and distributed by the Association of Analytical Chemists Suite 400, 2200 Wilson Blvd, Arlington, VA 22201-3301.

"BHI broth" means brain-heart infusion broth.

"Graduated Mass Ladder" means a marker solution comprising Water (CAS No. 7732-18-5), 96.6276 Weight %: Polyepichlorohydrin (CAS No. 26837-85-8, 2,5 Weight % available as "FICOLL, Type 400-DL" from Sigma Chemical Company, Dorset, England; Tris (CAS No. 77-86-1) 0.54 Weight % available from Fisher Scientific; Boric Acid (GAS No. 10043-35-3) 0.27 %; Ethylenediaminetetraacidic Acid (EDTA) (CAS No. 60-00-4) 0.029 Weight %; Xylene Cyanol FF (CAS No. 2650-17-1) 0.016 Weight %; Sodium Dodecylsulfate (SDS) (CAS No. 151-21-3) 0.016 Weight % and DNA 0.0014 Weight % from Life Technologies, Incorporated, Gaithersburg, MD.

"Lysis Buffer" means a solution of water (CAS No. 7732-18-5) 99.51 Weight %; Potassium chloride (CAS NO. 7447-40-7) 0.208 Weight %; Tris (CAS NO. 77-86-1) 0.12 Weight %; Magnesium Chloride (CAS No. 7791-18-6) 0.061 Weight % and TRITON X-100™ (Octylphyenoxypolyethoxyethanol nonionic surfactant) (DP-17-67-8) available from Union Carbide, 0.1 Weight % to which Proteinase K is added in the protocol (available from Behringer Mannheim, Indianapolis, IN). The solution without Proteinase K is shipped as DDS-24 Lysis Reagent by E. I. du Pont de Nemours and Company, Wilmington, DE.

"Tracking Dye" means a solution of water 84.61 Weight %; FICOLL 15.0 Weight %; EDTA 0.19 Weight %; SDS 0.1 Weight %; Xylene Cyanol FF 0.1 Weight % and EDTA 0.19 Weight % available from E. I. du Pont de Nemours and Company, Wilmington, DE 19898.

"PAGE" means polyacrylamide gel electrophoresis.

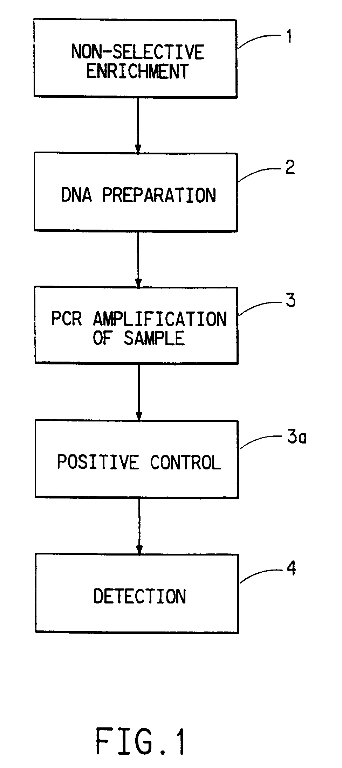

Figure 1 shows the method of the invention which comprises three steps following conventional non-selective enrichment: DNA preparation which results in a solution which can represent the total volume used to hydrate the tablets (thus allowing the single dose addition), PCR amplification of both sample and control using tabletted reagents wherein the control is insensitive to the absence (or presence) of the target DNA, and detection. Applicants use either homogeneous detection or gel electrophoresis for detection preferring the latter.

A graduated mass ladder is electrophoresed on each gel. This reagent contains DNA fragments of six different sizes such that a ladder representing a range of size and mass loading results upon electrophoretic separation yielding markers stepped in specific locations and of varying intensity. See step 11 immediately below. Following electrophoresis and staining of the gel in the described method and visualization of all six bands in the lane containing the DNA ladder indicates sufficient sensitivity of the detection system.

A protocol for the definitive detection process of the invention follows. It is in sequential steps.
1. Non-selectively enrich the food matrix under consideration for at least 20 hrs according to BAM (or other reference method(s)) protocol.
2. Dilute the non-selective enrichment broth by 1:10 factor using BHI broth (i.e., 1 mL pre-enrichment broth into 9 mL BHI broth), first pre-warming the BHI broth to 37 °C.
3. Incubate the diluted broth for 3 hrs at 37 °C.
4. Transfer 5 uL of the diluted non-selective enrichment broth into a 2 mL polypropylene screw cap tube (with O-ring for sealing) containing 195 uL lysis buffer solution, first pre-warming the lysis buffer solution to room temperature before use. Mix the solutions.
5. Incubate the lysis tube for 20 min at 37 °C in a water bath.
6. Heat the lysis tube for 10 min at 95 °C in a second water bath.
7. Transfer 50 uL of lysate into a sample PCR tube containing a *Salmonella* PCR tablet. Use a clear tube to distinguish it as the test sample.
8. Transfer an additional 50 uL lysate into a PCR tube containing a positive control tablet. We use a pink tube to distinguish it as the control. Note: The lysate will dissolve the PCR tablets with no mixing as long as the tablets are well wetted; avoid air trapped underneath the tablet and lysate solution.
9. Place the PCR tubes into a Perkin Elmer Cycler (Model 9600) and initiate the amplification program starting with 2 min and 15 sec at 94 °C followed by 35 cycles of a 2 temperature protocol: 15 sec. at 94 °C and 3 min at 72 °C. After the last cycle at 72 °C there is a 7 min holding period at 72 °C.
10. Conduct PAGE (polyacrylamide gel electrophoresis) as follows using either of the two listed paths which are equivalent:
   a) VERSION I. Add 1.7 uL of Tracking Dye and 8.3 uL PCR product into an empty 500 uL microcentrifuge tube and mix by centrifugation.
   b) VERSION II. "add-10-load-10": add 10 uL Tracking Dye into the PCR tube containing the amplified sample and mix the solution by repeated uptake and expulsion of the solution for a total of four cycles.
11. Pipette 10 uL of graduated mass ladder into lane 1 of the gel.
12. Load (by pipette) 10 uL of the PCR/tracking dye mixture into the other lanes of the gel.
   Note: Each food sample test is represented by 2 gel lanes; one for the sample tablet and one for the positive control tablet.
13. Follow standard PAGE separation and detection procedures. Failure to detect the control product indicates a test failure for negative results in the test well. Failure to detect the control product with a positive test sample is considered a positive test for screening purposes. Such a case would likely be a result of a small degree of inhibition and a high level of target DNA in the test sample to produce sufficient product. Electrophoresis protocols other than PAGE may be adapted to this step as is well-known to those skilled in the art. For example, pre-stained gels containing a nucleic acid-specific dye such as ethidium bromide may be used. This allows visualization of the band promptly after the electrophoresis step.

The deficiency of the prior art, the absence of a control and simple control procedure which will work in the presence of a positive target in the analysate, is removed by using the DNA construct of the invention in a parallel reaction which offers the following benefits:
- is able to be amplified using a single primer;
- the amount of the amplification product is independent of any DNA contained in the sample;
- can be tabletted for ease of use and high degree of reproducibility in both manual and automated test procedures.
- can be used with homogeneous detection, i.e., without separation of product DNA from reactants.
- avoids competing reactions by using separate test vessels for the suspected target and for the control.

The control DNA of the invention, a construct, is comprised of a DNA vector (pUC18) containing an insertion of the *Salmonella* specific DNA sequence. The sequence pUC18 is ligated with the *Salmonella* fragment extending from base pair 35 to base pair 786 to form the recombinant pUC18 with 35 and 761 priming sites in the plasmid. Insertion of primer 761 sequence within the 35 primer site then forms a further-modified, recombinant pUC18 containing two 761 priming sites.

The resultant plasmid is able to be amplified using only primer 761 while the natural *Salmonella* target sequence requires both the 35 and 761 primers. In this way the positive control reaction which is run along with the test and includes test analysate in the control reaction well will not amplify *Salmonella* DNA exponentially if present. Instead the control reaction produces only product using the plasmid as target and, therefore, gives a constant product level unaffected by the presence or absence of target DNA.

The construct, then, effectively is the *Salmonella* specific DNA fragment with two ends that differ only in that they are reverse compliments of each other. That is to say the priming sequences are complimentary in opposite orientation. "Effectively" is used because in actuality the insertion at the 5' end is within the end sequence and so, in fact, is not quite at the end but functions as though it were.

This is the sequence cloned into pUC18:

The positive control tablet is composed of:

| Trehalose | 90.15 Weight % |
|---|---|
| Carbowax | 9.82 |
| dATP (deoxynucleotidetriphosphate) | <0.10 |
| dCTP (deoxynucleotidetriphosphate) | <0.10 |
| dGTP (deoxynucleotidetriphosphate) | <0.10 |
| dTTP (deoxynucleotidetriphosphate) | <0.10 |
| 761 Primer | <0.001 |
| Plasmid DNA | <0.001 |
| *TAQ*® polymerase | <0.001 |

The test tablet is composed of:

| Trehalose | 90.15 Weight % |
|---|---|
| Carbowax | 9.82 |
| dATP (deoxynucleotidetriphosphate) | <0.10 |
| dCTP (deoxynucleotidetriphosphate) | <0.10 |
| dGTP (deoxynucleotidetriphosphate) | <0.10 |
| dTTP (deoxynucleotidetriphosphate) | <0.10 |
| 761 Primer | <0.001 |
| 35 Primer | <0.001 |
| *Taq*® polymerase | <0.001 |

A negative control tablet, if desired, is composed of:

| Trehalose | 90.15 Weight % |
|---|---|
| Carbowax | 9.82 |
| dATP (deoxynucleotidetriphosphate) | <0.10 |
| dCTP (deoxynucleotidetriphosphate) | <0.10 |
| dGTP (deoxynucleotidetriphosphate) | <0.10 |
| dTTP (deoxynucleotidetriphosphate) | <0.10 |
| 761 Primer | <0.001 |
| 35 Primer | <0.001 |

As mentioned above, both control and test tablets are prepared using the "snow gun process" as set forth in Fermani et al. (U.S. Patent No. 5,475,984) . The process uses a cryogenic liquid for producing frozen particles of a liquid product in a housing which comprises the steps of: (a) introducing the cryogenic liquid into the housing in an annular, downward direction creating a substantially continuous downwardly directed circumferential wall of cryogenic liquid, defining an interior entrapment zone; and (b) introducing droplets of the liquid product into the entrapment zone, whereby the cryogenic liquid freezes the liquid product droplets to produce frozen particles.

In use, a sample is subjected to the test PCR procedure in parallel with a control containing the construct as well as the sample. If the control shows amplification, there is positive indication that the procedure has been effective regardless of the positive or negative results attained in the parallel test.

It is well known that sample matrix components, including food, can cause inhibition of PCR and therefore a resulting decrease in product formation and signal. On the other hand, the presence of certain food components in the PCR reaction have also been found to result in the opposite result: enhancement of the signal when fluorescent dye detection is employed. In non-homogeneous detection systems, such enhancement may not be noted and would not cause difficulty with analysis. However, in a homogeneous detection system, signal enhancement in the absence of a proper control could lead to false positive results. Use of the control as described herein would eliminate such false positive results. Moreover, by calibrating the level of response in the control, it is possible to evaluate and compensate for any suppression or enhancement of the reaction in the test caused by extraneous material such as is found in many food-derived matrices.

To repeat, this invention concerns a control reactant, its method of making, and its method of use in PCR. By using the control reactant in parallel with the test, assurance is provided that the processing cycle was operative. The reactant, preferably in tablet form, comprises 1) a synthetic construct of a characterizing DNA sequence with end sub-sequences that are complements of each other along with a single primer complimentary to the sub-sequences, 2) an indicator (an intercalating dye which may be added after PCR cycling), and 3) a stabilizer (trehalose as described in Bollin, Jr., et al., U.S. Patent 4,762,857) more preferably processed generally according to the teachings of U.S. Patent 5,307,640 (Fawzy et al.) as improved in U.S. Patent No. 5,475,984 using a spray-freeze, "snow-gun" process, both specifications incorporated by reference herein.

In use (by the single dose methodology), the analysate in solution is subjected to lysis and the resultant solution is introduced into two vials, one with test ingredients and the other with control ingredients.

A positive result in the control vial means that the process worked and the test result in the test vial may be given credence in detecting the target DNA. The combinations of results possible, and the conclusions stemming from these results, have been discussed above.

By calibrating the control reaction and because analysate is provided in the control vial as well as the test vial, a correction can be made for the effects of fluorescence enhancement or suppression by extraneous matter from the original sample. The presence of extraneous matter having an active effect on test results is quite common in practical applications of PCR-based testing, including applications involving food products.

The use of the construct and single primer in a control well allows introducing analysate into the control well without amplification of any target bacteria DNA in that analysate which might yield an invalid control result. At the same time, the presence of the analysate in the test well yields an indicator reaction permitting correction to the result seen in the parallel test well.

### EXAMPLE 1

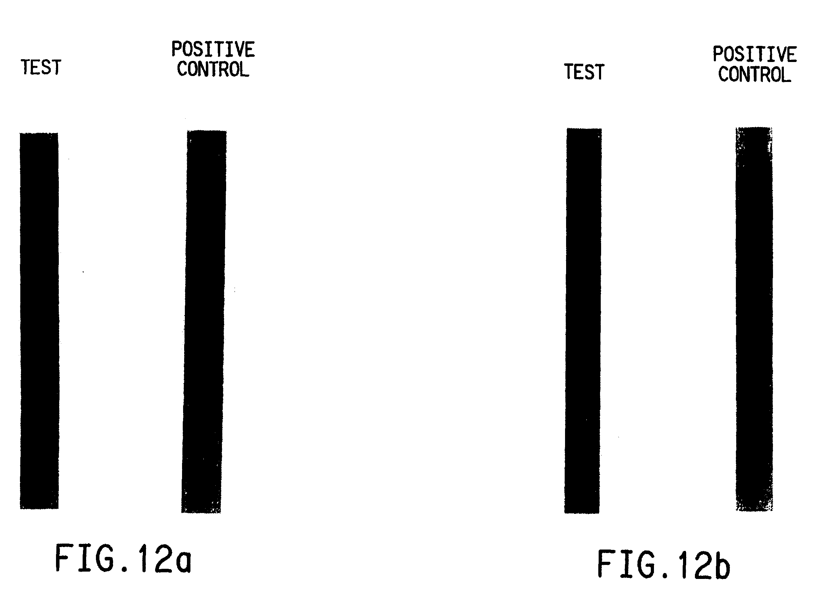

Example 1 was run to demonstrate practical use of the invention in determining the presence of a pathogen in a food sample.
1. 25 g of food (ground beef purchased at a local supermarket) was placed into a flask containing 225 mL of lactose broth. The mixture was homogenized (in a stomacher) and then incubated at 37 °C for 24 h (± 2 h is permitted in the protocol).
2. Following the 24 h non-selective enrichment of step 1, a piece of Porex™ high density polypropylene (HDP) (Porex Technologies, Fairburn, GA, Catalog No. 6949) was placed into the flask. The flask was then swirled to completely wet the piece of Porex™ HDP with the non-selective enrichment media.
3. The flask was then incubated at 37 °C for 20 min.
4. Following the incubation, the Porex™ HDP was then removed from the media and placed in a 15 mL tube that contained 10 mL of wash buffer (10 mM Tris-HCl, 28 mM KCl and 3 mM MgCl₂, pH=8.3). The tube was capped and inverted 25 times to completely wash the Porex™ HDP.
5. The Porex™ HDP was removed from the 15 mL tube and excess wash buffer was removed by placing the piece of Porex™ HDP on Whatman Number 1 filter paper for 5 sec.
6. The Porex™ HDP was placed in a 2 mL screw cap tube. A 200 uL aliquot of lysis reagent (10 mM Tris-HCl, 28 mM KCl and 3 mM MgCl₂, pH=8.3 containing 0.25 mg of Proteinase K per mL) was added to the tube. The tube was capped and placed in a 55 °C water bath for 20 min. (The lysis reagent can be incorporated in a tablet.)
7. Following the 55 °C incubation, the tube was placed at 95 °C for 10 min.
8. 50 uL of lysate obtained from processing the Porex™ HDP was used to hydrate a *Salmonella* specific PCR reagent tablet (described in detail below) in a 0.2 mL reaction tube.
9. A second 50 uL aliquot of the same lysate was used to hydrate a control PCR tablet (described in detail below) in a second 0.2 mL reaction tube.
10. Both reaction tubes were thermally cycled under the following conditions:
   a. 94 °C, 2 min 1 cycle
   b. 94 °C, 15 sec
      65 °C, 1.5 min
      72 °C, 0.5 min
      Sequence b. was repeated for a total of 35 cycles
   c. 72 °C, 7 min 1 cycle
   d. 4 °C and held at this temperature until used.
11. Following the thermal cycling of the samples, the DNA amplified products were separated by gel elctrophoresis on a 4% polyacrylamide gel (29:1). The electrophoresis was run using a 0.5x TBE buffer (45 mM Tris-base, 45 mM Boric Acid and 1 mM EDTA) at a constant voltage of 100 volts for 30 min.
12. The separated DNA bands (see Figures 12a and 12b showing replicated tests) were stained in a solution of ethidium bromide 0.1 ug/mL) for 15 min. and then visualized by placing the gel on a UV transilluminator (such as a FOTO UV 300 available from Fotodyne, Inc., New Berlin, WI).
13. A determination of whether the sample contained *Salmonella* was made based on the following criteria:
   a. A band corresponding to 750 bp in both sample and control lanes -- indicating that the sample was positive for *Salmonella.*
   b. No band in the sample lane and a band corresponding to 750 bp in the control lane -- indicating that the sample was negative for *Salmonella.*
   c. A band in the sample lane and no band on the control lane -- in this instance, the PCR reaction is compromised due to a presumed matrix effect such that the typical 104 CFU/mL sensitivity representative of the control reaction was not achieved. The positive result in the test lane indicates that the sample contained *Salmonella* at a higher concentration than the sensitivity limit of 104 CFU/mL. Result indicated that the sample was positive for *Salmonella.*
   d. No band corresponding to 750 bp in either the sample lane or the control lane -- no result can be reported. Due to either a chemical or mechanical (including thermal) abnormality the PCR process was compromised such that a sample containing 104 CFU/mL would not have been sufficiently amplified to be detected.

Analysis of the bands shown in Figures 12a and b revealed that the 750 bp band was present in the sample lane and control lanes. According to the criterium 13a. above, the functionality of the protocol had been demonstrated and the sample was positive for the organism.

Note that in steps 2 - 4 in this example above the incubated organisms were separated using Porex™ HDP. Applicants prefer the method of the preferred protocol in which a 1:10 dilution and regrowth is used (see steps 2 -3 of the protocol above).

### EXAMPLE 2

This example shows the use of the preferred dilution/regrowth method of sample preparation with a food product.
1. Black pepper and frozen yogurt samples were non-selectively enriched by standard methods (BAM-FDA)
2. Following incubation (step 3 of Example 1 above) a portion of the non-selective enrichment was spiked with *Salmonella typhimurium* at a concentration of 104 cells per mL.
3. One mL portions of the spiked and unspiked non-selective enrichment were added to separate 15 mL screw cap tubes containing 9 mL of brain heart infusion broth (BHI).
4. The tubes were incubated at 37 °C for 3 h.
5. Five microliter volumes were removed from each tube and added to 195 microliters of lysis reagent (10 mM Tris-HCl, 28 mM KCl and 3 mM MgCl₂, pH=8.3 containing 0.25 mg of preteinase K per mL and 0.1% Triton X-100) in 2 mL screw-cap tubes. The tubes were incubated at 37 °C for 20 min, then 95 °C for 10 min.
6. Fifty microliter portions of each lysate sample prepared above were used to hydrate PCR sample tablets and PCR control tablets.

The procedure followed was as in Example 1 steps 10-13.

Analysis of the bands showed the same 750 bp bands as in Figures 12a and b of Example 1.

| CONTROL, TABLET FORMULATION | | |
|---|---|---|
| REAGENT | QUANTITY/TABLET | QUANTITY/LOT |
| | | (50,000 tablets) |
| deoxy nucleotides (25.0 mM) | 0.32 uL | 16.0 mL |
| primer 33-26-rc761 (20.0 um) | 0.09 uL* | 4.5 mL |
| control DNA | 0.02 uL | 1.0 mL |

| | (app. 104 copies) | |
|---|---|---|
| *Taq*® polymerase | 1.5 units | 75,000 units |
| trehalose | 6.608 mg | 330.4 g |
| carbowax | 0.72 m | 36 g |

| | | |
|---|---|---|
| *Range 0.03-0.90 uL | | |

### PREPARATION OF CONTROL, PCR TABLET

1. The trehalose dihydrate and carbowax were dissolved in 750 mL of autoclaved deionized water.
2. The deoxy nucleotides, primer, control DNA, and *Taq*® polymerase were added to the solution.
3. The solution was adjusted to the final weight of 1357 g with autoclaved deionized water.
4. The solution was filtered through a 5 um cartridge.
5. The solution was then processed generally according to the teachings of U.S. Patent 5,307,640 (Fawzy et al.) as improved in U.S. Patent No. 5,475,984, a process dubbed a "snow-gun process." The solution was sprayed into a liquid nitrogen chamber at a reagent spray rate of 125 mL/min.
6. The frozen blend was collected in the tray at the end of the chamber due to gravity.
7. The frozen blend was then freeze-dried in a freeze drier (such as the GT6, available from Finn Aqua of Germany). The freeze drying program consisted of primary drying at a product temperature of -40 °C and a chamber pressure of 50 micron for 50 h. Secondary drying was done at 25 °C for 20 h.
8. The freeze dried blend was then sized through a 30 mesh screen.
9. The sized blend was then tabletted using a 3/32 inch tool.

| SAMPLE TABLET FORMULATION | | |
|---|---|---|
| REAGENT | QUANTITY/TABLET | QUANTITY/LOT |
| | | (50,000 tablets) |
| deoxy nucleotides (25.0 mM) | 0.32 uL | 16.0 mL |
| primer 33-26-rc761 (10 um) | 0.10 uL** | |
| primer 33-26-35 (10 um) | 0.10 uL** | 9.0 mL |
| *Taq*® polymerase | 1.5 units | 75,000 units |
| trehalose | 6.608 mg | 330.4 g |
| carbowax | 0.72 mg | 36.0 g |

| | | |
|---|---|---|
| **Range 0.06-1.80 uL | | |

### PREPARATION OF SAMPLE PCR TABLET

1. The trehalose dihydrate and carbowax were dissolved in 750 mL of autoclaved deionized water.
2. The deoxy nucleotides, primer and *Taq*®
polymerase were added to the solution.
3. The solution was adjusted to the final weight of 1357 g with autoclaved deionized water.
4. The solution was filtered through a 5 um cartridge.
5. The solution was then processed generally according to the "snow gun" process as above. The solution was sprayed into a liquid nitrogen chamber at a reagent spray rate of 125 mL/min.
6. The frozen blend was collected in the tray at the end of the chamber due to gravity.
7. The frozen blend was then freeze-dried in a freeze drier (such as GT6 available from Finn-Aqua of Germany). The freeze drying program consisted of primary drying at a product temperature of -40 °C and a chamber pressure of 50 micron for 50 h. Secondary drying was done at 25 °C for 20 h.
8. The freeze dried blend was then sized through a 30 mesh screen.
9. The sized blend was then tabletted using using a 3/32 inch tooling.

Following are examples which describe the procedures followed in constructing the control plasmid and confirming its utility for the test procedure described above.

### EXAMPLE 3

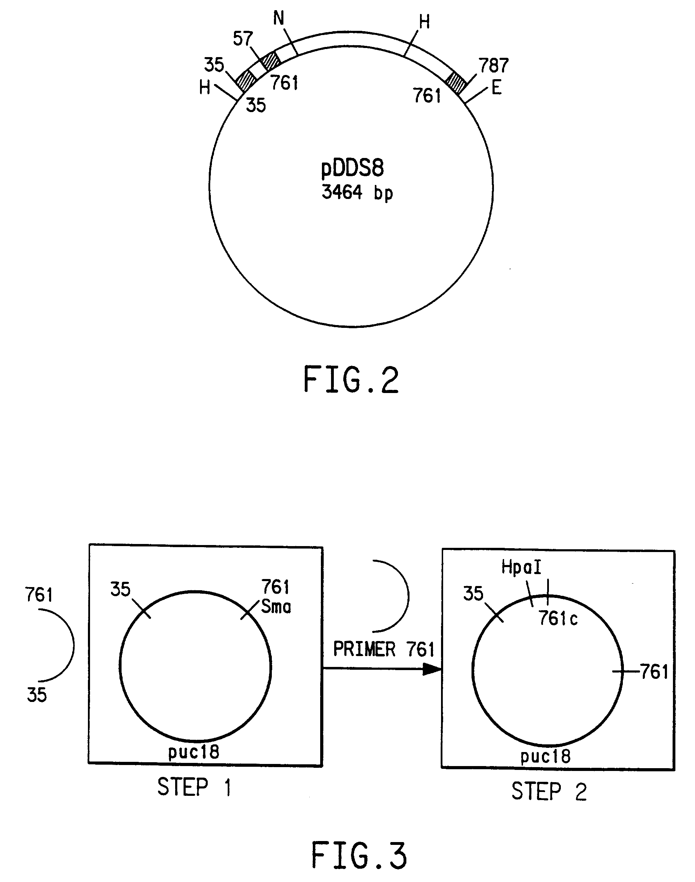

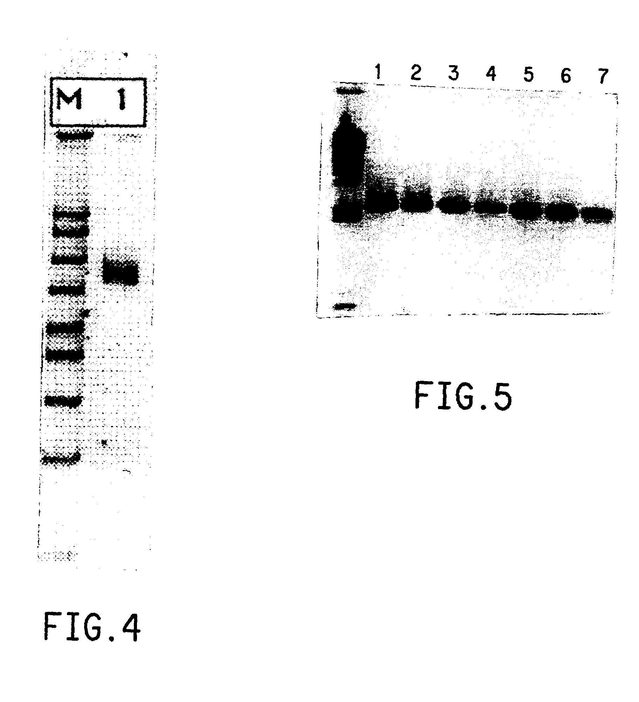

Example 3 was run to construct a control DNA plasmid, as shown in Figure 2, by cloning a *Salmonella* typhimurium PCR product. The specific object was to clone 0.7 kb PCR product into the SmaI site of pUC18, then insert the complement of 761 (761c) primer sequence near 5' end (35 end) of the cloned fragment (primers, both 761 and 35, were prepared by Research Genetics, Huntsville, AL on a custom order). In this way the final product could be amplified with a single 761 primer to generate a near full length product. Steps 1 and 2 are shown in Figure 3 and the results are shown in Figure 4.

Procedural steps were as follows:
i) linearized pUC 18 with SmaI
ii) gel purified PCR product DNA
iii) ligated above and transformed DH5alpha
iv) cut clone with HpaI
v) annealed 761c with 761
vi) ligated iv and v
vii) transformed material, selected for clones amplified with 761 primer alone.

pUC 18 was prepared using 5 uL pUC18 DNA (Life Technologies Inc.), 2 uL React 4 buffer (Life Technologies Inc.), 11 uL H₂O and 2 uL SmaI (Life Technologies Incorporated). The 20 uL mixture was heated for 2 h at 37 °C. Then 4 uL of 50 mm EDTA was added. The procedure was repeated and the two digestions were pooled. 4 uL of the resultant product was gel analyzed as follows:
i) ran 20 uL/lane on acrylamide gel (see Fig. 3)
ii) cut out band on transilluminator
iii) chopped up piece in 1.5 uL tube with pipette tip
iv) incubated at 4 °C overnight in TE buffer (approximately 20 h)
v) spun through a 0.65 um Ultrafree cartridge (Millipore)
vi) Ethanol precipitated
vii) analyzed on acrylamide gel

Three ligations were made:
#1. puC18 SmaI + unpurified PCR product
#2. puc18 SmaI + purified product
#3. puc18 SmaI

These were composed as listed below:

| #1 | #2 | #3 |
|---|---|---|
| 1 uL puc18 SmaI | 1 uL puc18 SmaI | 1 uL puc18 SmaI |
| 1 uL unpurified prod | 1 uL purified prod | 2 uL 5x buffer |
| 2 uL 5x buffer | 2 uL 5x buffer | 6 uL 5x H₂O |
| 5 uL dH₂O | 5 uL H₂O | 1 uL Ligase |
| 1 uL Ligase | 1 uL Ligase | --- |

The three 10 uL aliquots were incubated for 4 h at 15 °C and transformations were prepared in *E. coli* DH5alpha and analyzed on plates as listed below:

| TRANSFORMATIONS | PLATES |
|---|---|
| i) 3 uL Ligation #1 | |
| ii) 3 uL Ligation #2 | 100 ug/mL Ampicillin (50 mg/mL stock) |
| iii) 3 uL Ligation #3 | 50 uL x-Gal (20 mg/mL) |
| iv) 5 uL Control puc18 plasmid | 4 uL IPTG (200 mg/mL) |
| v) no DNA | |
| vi) unligated pUC 18 SmaI | |

Seven colonies were picked from ampicillin 100 ug/mL plates of transformation ii) to check for inserts. These were all white or faint blue colonies. A Hind III digest was carried out. "Wizard" minipreps of the seven colonies were prepared according to the Promega directions and 5 uL of each was cut with Hind III. The result was that all clones apparently were the same size indicating no inserts (see Figure 5); therefore, additional screening was done.

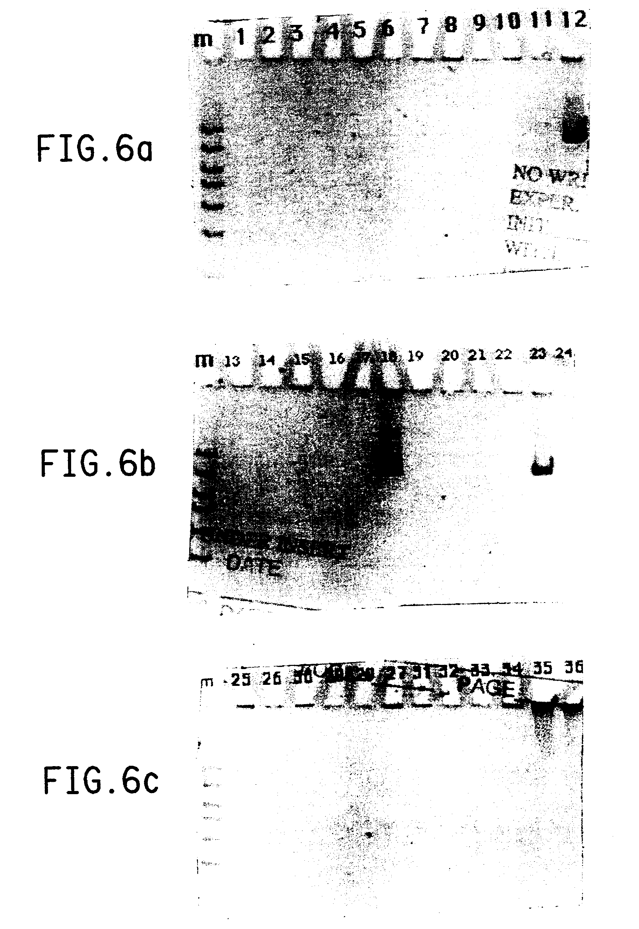

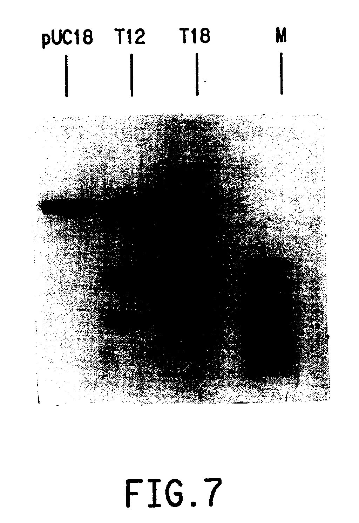

Thirty six colonies were picked from a pooled and replated group of colonies and transferred to a fresh plate. Using a loop, a very small amount of the colonies was picked and suspended in about 500 uL of lx PCR buffer. Each suspension was heated at 95 °C for 10 min and each of the clones was amplified with sample tablets using PCR. The results are shown in Figures 6a, b and c. Clones 12 and 18 were selected for further analysis (and designated T12 and T18) as these appeared to have inserts. Analysis was done by EcoRI/Hind III digests. 5 uL each of minipreps (Wizard miniprep, Promega) was cut with EcoRI and Hind III in React 2 buffer. These were checked for insert on 1% agarose gel using as a marker Biomarker Low (BioVentures). The electrophoresis was about 1 h at 100 volts. See Figure 7 for the result. Proper sized inserts were seen in both clones corresponding to a Hind III site at 581 bp as predicted. See Table I for restriction sites on insert.

**TABLE I**

| Restriction Enzyme Report for 3,3 sal 001-811 Length of 3,3 sal 001-811 (linar): 811 bp; Simple Restriction from: 1 to: 811; | | | | |
|---|---|---|---|---|
| Enzyme | Site | Position (Fragment length) | | |
| Ase I | AT'TAAT | 166 (166) | | |
| Ban I | G'GYRCC | 183 (183) | 195 (12) | 312 (117) |
| BsaA I | YAC'GTR | 520 (520) | | |
| BsaH I | GR'CGYC | 184 (184) | 196 (12) | |
| BsaJ I | C'CNNGG | 214 (214) | 398 (184) | 446 (48) |
| | | 494 (48) | | |
| BsiE I | CGRY'CG | 149 (149) | | |
| BspH I | T'CATGA | 705 (705) | | |
| BssH II | G'CGCGC | 726 (726) | | |
| Dsa I | C'CRYGG | 398 (398) | | |
| Hae II | RGCGC'Y | 79 (79) | 187 (108) | 199 (12) |
| | | 494 (295) | | |
| Hinc II | GTY'RAC | 57 (57) | | |
| Hind III | A'AGCTT | 581 (581) | | |
| Hpa I | GTT'AAC | 57 (57) | | |
| Hsu I | A'AGCTT | 581 (581) | | |
| Kas I | G'GCGCC | 183 (183) | 195 (12) | |
| Nar I | GG'CGCC | 184 (184) | 196 (12) | |
| Nla IV | GGN'NCC | 185 (185) | 197 (12) | 314 (117) |
| Nsi I | ATGCA'T | 112 (112) | | |
| NspB II | CMG'CKG | 230 (230) | 400 (170) | |
| Nsp II | CDGCH'C | 139 (139) | 282 (143) | |
| Nsp III | C'HCGVG | 138 (138) | | |
| Sac II | CCGC'GG | 401 (401) | | |
| Sfc I | C'TRYAG | 97 (97) | | |
| Sst II | CCGC'GG | 401 (401) | | |
| Sty I | C'CWWGG | 214 (214) | | |

### EXAMPLE 4

Example 4 was run to clone the 761 priming site into T12, the intent being to insert annealed 761 and 761c (compliment of 761) into the Hpa I site at 57 bp in the insert DNA (see Table II).

The following steps were carried out in sequence:
i) Digest T12 with HpaI
ii) Dephosphorylate above
iii) Anneal primer 761 with 761c
iv) Ligate vector T12 with annealed primers
vi) Transform DH5a selecting for ampicillin resistance
vii) Screen for inserts of primer by amplification with 761 primer only

The digests of T12 and T18 with HpaI were:

| T12 | T18 |
|---|---|
| 34 ul DNA | 10ul DNA 6 ul H₂O |
| 4 uL 10xbuffer | 2 uL 10x buffer |
| 2 uL HpaI | 2 uL HpaI |

These were held at 37 °C for 3 h.

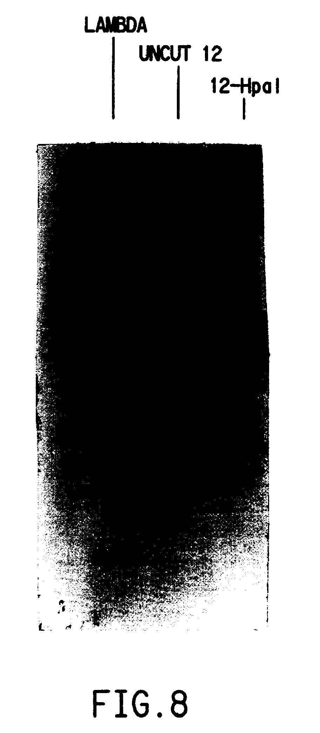

Figure 8, an electrophoresis of T12 only, shows that cutting took place. T18, was gel separated also (but not shown in the figure) and the digest looked cleaner and was used in ligation as follows with primer prepared by mixing primer 761 with its compliment, 25 uL each at 0.05 ug/uL, then heating at 94 °C for 2 min followed by a ramp to 37 °C over 30 min, then an 8 minute to 4 °C in a Perkin Elmer cycler:

| CONTROL | TEST |
|---|---|
| 2 uL T18 HPAI | 2 uL T18 HpaI |
| 2 uL 5xBuffer | 2 uL 5x buffer |
| | 1 uL annealed primer (1/5x dilution) |
| 5 uL H₂O | 4 uL H₂O |
| 1 uL Ligase | 1 uL Ligase |

These were held at 15 °C for 4 h.

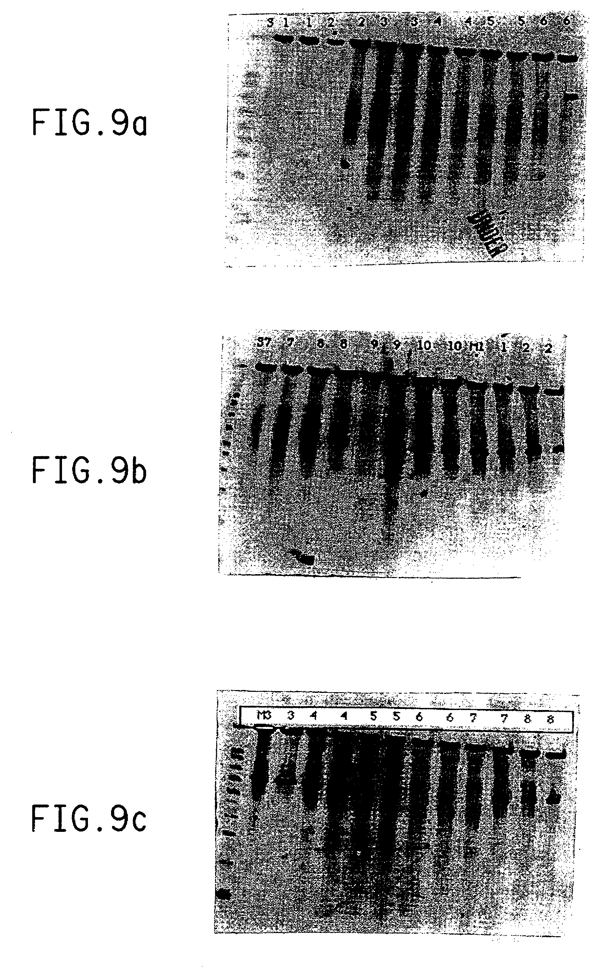

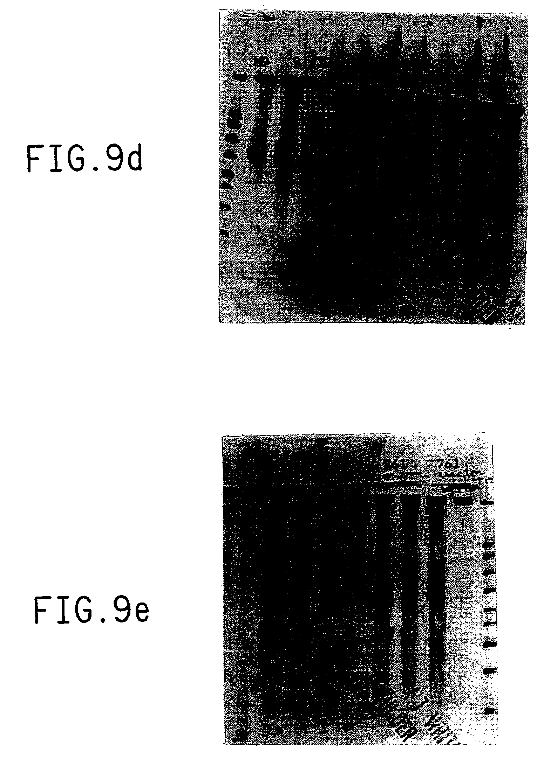

Three uL of each ligation was used to transform DH5ALPHA selecting for ampicilin resistance and loss of b-galactosidase activity. Ten colonies were picked from the test ligation and the remainder of the plate was pooled into 10 mixes. Lysates from all samples were amplified using only 761 primer. Also amplified were T12 vector and negative and positive controls. Included were amplifications with two primer tablets of T12 and negative and positive control DNAs. The results are shown in Figures 9a-e. S1 to S10 in Figures 9a and 9b are single colony isolates. In Figures 9b, c and d, m1 to m12 are mixes of about 20 colonies. Figure 9e shows controls. S8 and S10 were positive for a single 761 primer amplification as were all of the mixes.

### EXAMPLE 5

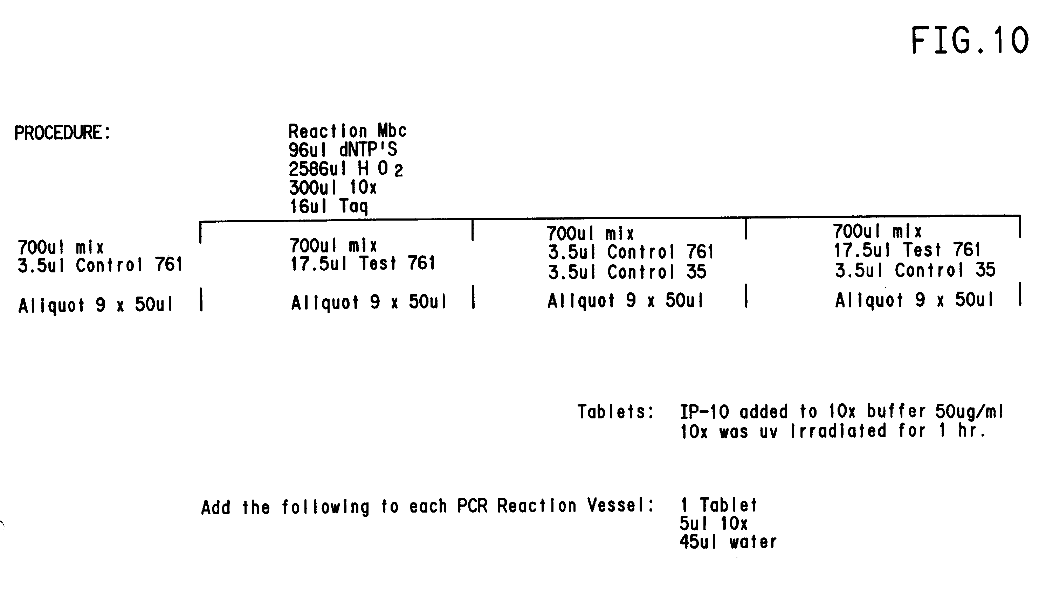

Example 5 was run to test the performance of the control DNA, to confirm its utility as a control and judge its suitability for later incorporation in a control tablet. The test was run according to the protocol shown in Figure 10 with the following experimental parameters:
- EXPERIMENT:: Positive Control Plasmid Testing
- PURPOSE:: To test the performance of the newly prepared positive-control DNA we will eventually use in a positive control tablet
- MATERIALS:: S-8 MiniPrep purified
S-8 and S-10 Lysates
PUC 18 (internal ref. I)
M1 Lysate - Prep. prior to S-8 Mini-Prep
Primers:
761 Test (Internal Ref. II)
761 Control (8/10/93 diluted 1:400 = -2uM)
35 Control 4/28/93 (10uM diluted 1:10 to 1uM)
dNTP's: 6/15/93 5mM
H2O: HPLC aliquoted and autoclaved
10x: (Internal Reference IIa)

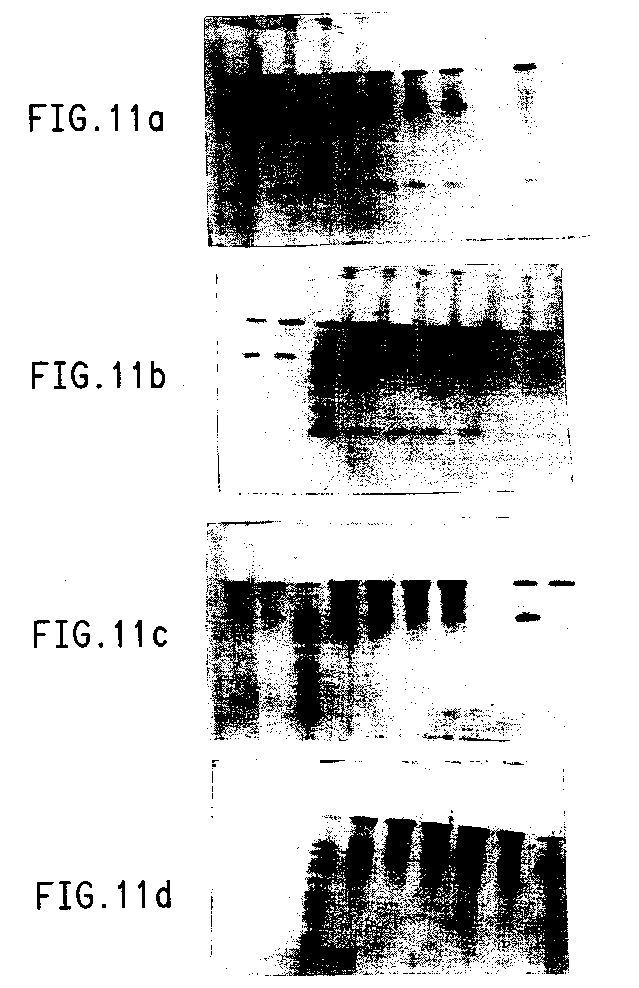

The results are shown in Figure 11. The lanes in this figure were as listed below:

| | |
|---|---|
| 1 & 2 | S-8 Mini Prep purified |
| 3 | MW Marker |
| 4 & 5 | S-8 Lysates |
| 6 & 7 | S-10 Lysates |
| 8 | M1 Lysate |
| 9 | Puc 18 |
| 10 | Blank |

It was concluded that the control DNA was suitable and a candidate for tabletting.

### EXAMPLE 6

A test was run to demonstrate the utility of the control, in tablet form, for homogeneous detection. Homogenates of two foods, cocoa powder and powdered whole milk, were prepared by mixing 25 g of these foods with 225 ml of lactose broth medium. The homogenates were in some cases spiked with *Salmonella typhimurium* at approximately 100 cells per mL. All homogenates were incubated approximately 20 h at 37 °C after which samples of the foods were prepared for PCR by lysing the bacteria in a solution of proteinase K, triton X-100 and PCR buffer. Next, 50 uL aliquots of the lysates were placed in tubes containing either test PCR tablets or control PCR tablets prepared as described above. These were subjected to thermal cycling as described in Example 1. Following cycling, 5 uL portions of the reactions were analyzed by polyacrylamide gel electrophoresis (PAGE).

Also, the same samples were analyzed by homogeneous detection as follows. To 45 uL of reactions, 5 uL of 20 micromolar TOTO1 dye (obtained from Molecular Probes, Inc., Eugene, OR) was added, then incubated for 5 min at room temperature. Fluorescent measurements of the mixtures was accomplished using a fluorimeter with excitation wavelength of 513 nm and emission wavelength of 533 nm. The fluorescence (in arbitrary fluorescence intensity units) was above the threshold level of 50 and therefore the test was valid for the milk samples. The fact that the test tablet amplification gave a fluorescence of 358 which is significantly greater than the control fluorescence indicates a positive test result. This is verified by the presence of the DNA product shown by the gel electrophoresis, as can be seen, and is an accurate result since this sample was spiked with *Salmonella typhimurium.*

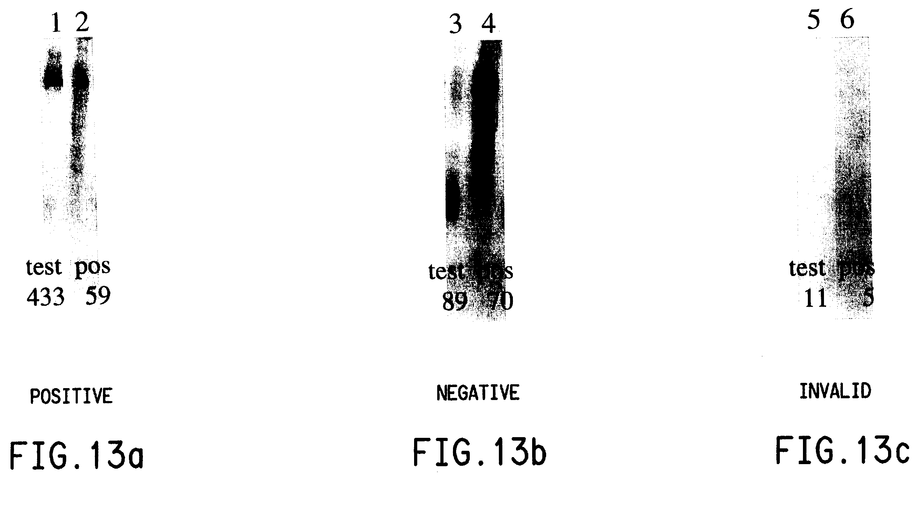

Figures 13a-c shows the results of the gel electrophoresis detection of the PCR reactions. Lanes 1 and 2 demonstrate a valid test with a positive result. Lanes 3 and 4 demonstrate a valid test with a negative result. The fluorescence of the control was above the threshold level of 50 and therefore the test was valid. The test tablet fluorescence, however, was not equal to or higher than the control indicating a negative test result. Again this was verified by the absence of a DNA product on the gel and was a correct result since this sample did not contain *Salmonella.* Finally, lanes 5 and 6 demonstrate an invalid test. The control tablet gave a fluorescence of 5 which is below the threshold of 50 and, therefore, the test is considered invalid. The accuracy of the homogeneous detection is again verified by the absence of DNA bands in lanes 5 and 6.

### EXAMPLE 7

### Evaluation of PAGE based definitive detection process

Example 7 was run to establish threshhold sensitivity according to the following:

*Salmonella* strains 1256 (*S. virchow),* 1261 (*S. newport),* (*S. hadar)* and 1231 were spiked into samples of ground beef, sausage, and ground pork purchased in a supermarket at a level of about 10E4/mL. This was done after the samples had been suitably prepared and subjected to a standard BAM twenty-four hour pre-enrichment procedure. This was followed by a 1/10 dilution and grow back in the original flask in BHI at 37 °C for 3 h, lysis, division into aliquots, each split into two with one portion for the PCR test and one for the PCR positive control and each test having three levels of dilution. PAGE based analysis followed. The aliquots were as follows:

| Fig. | Test | Sample | Innoculant |
|---|---|---|---|
| 14a | (a) | Ground Beef [gb] | -- Negative Control |
| 14a | (b) | [gb] | -- + 1256 (*S. virchow)* |
| 14b | (c) | [gb] | -- + 1261 (*S. newport)* |
| 14b | (d) | [gb] | -- + 1231 (*S. hadar)* |
| 14c | (e) | Sausage [gs] | -- Negative Control |
| 14c | (f) | [gs] | -- + 1256 (*S. virchow)* |
| 14d | (g) | [gs] | -- + 1261 (*S. newport)* |
| 14d | (h) | [gs] | -- + 1231 (*S. hadar)* |
| 14e | (i) | Ground Pork | -- Negative Control |
| 14e | (j) | [gp] | -- + 1256 (*S. virchow)* |
| 14f | (k) | [gp] | -- + 1261 (*S. newport*) |
| 14f | (1) | [gp] | -- + 1231 (*S. hadar)* |

Tablets used were internal reference II and Control, internal reference VI, direct protocol in 5 uL samples.

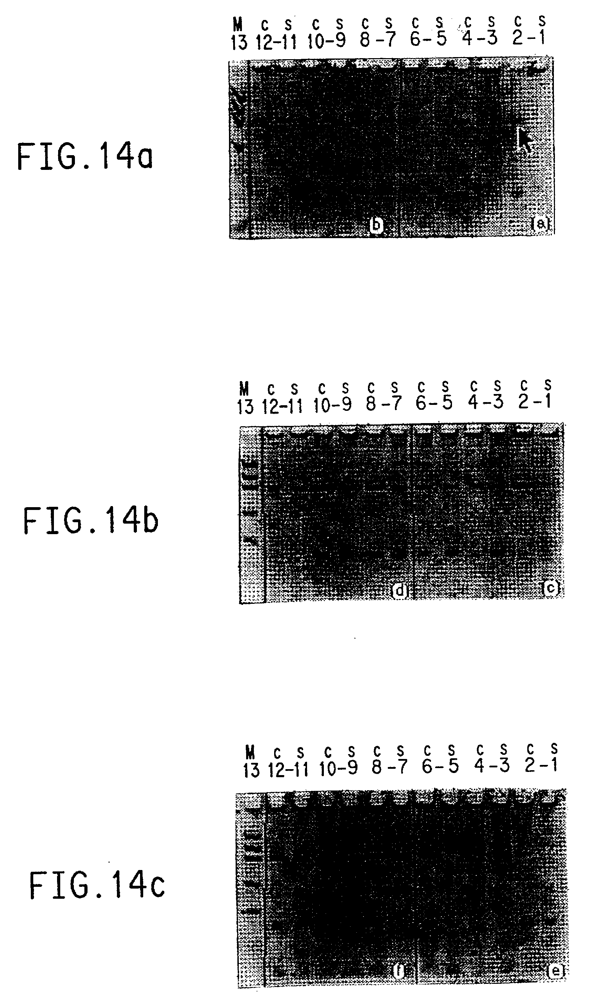

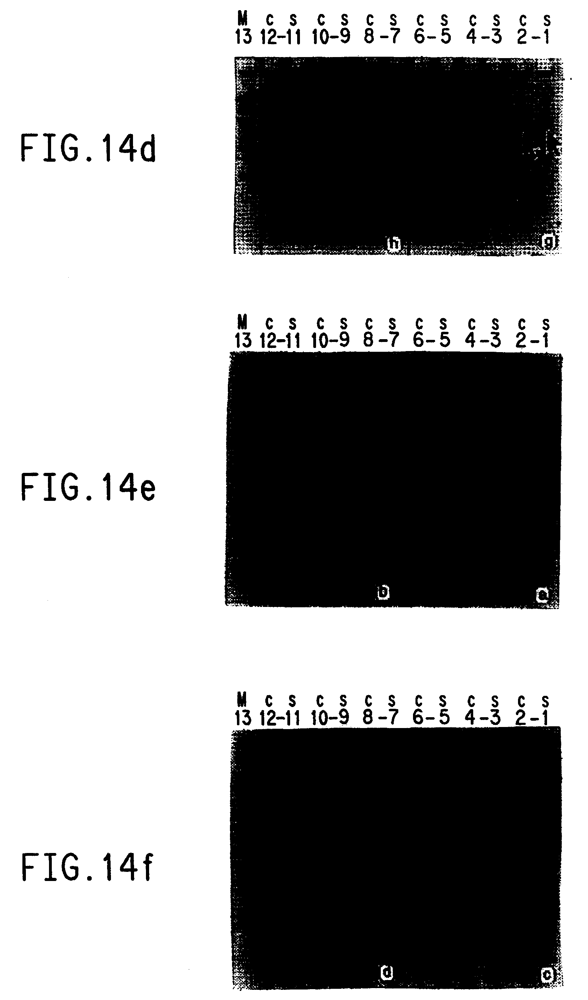

The results are shown in Figures 14a-f. Arrows denote positive bands. Applicants concluded that the threshhold sensitivity for these organisms is about 10E4.

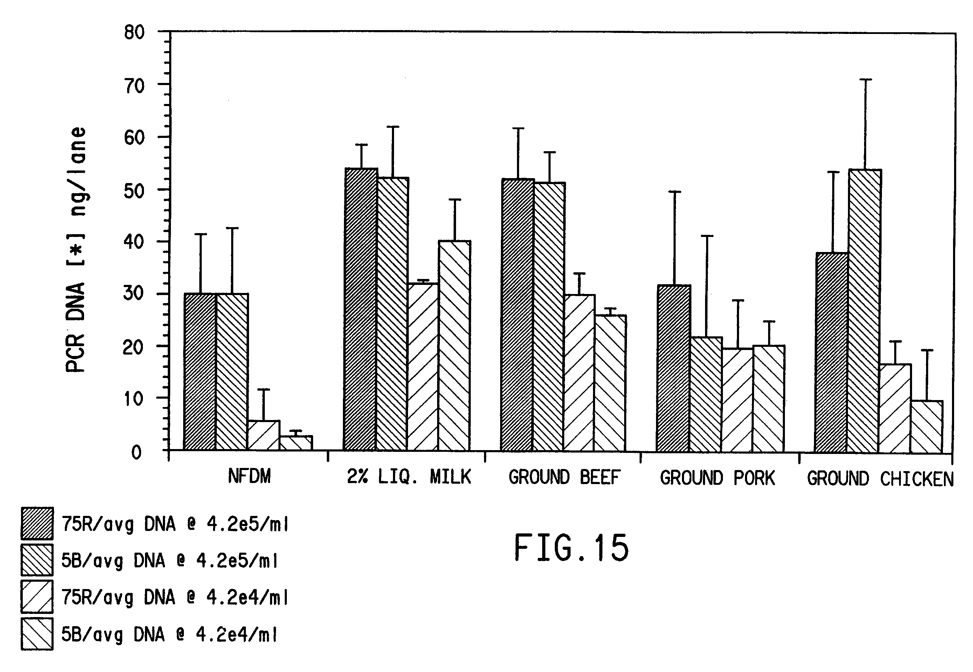

This was supported by a series of tests on the following: no-fat dried milk, 2 percent milk, ground beef, ground pork and ground chicken. Two lots of test reagents were used with only small differences in the results. Testing was done at 10E4 and 10E5 with typical confirming results shown in Figure 15 for *S. typhimurium.* A 10E4 threshold appears adequate for all these food matrices.

### EXAMPLE 8

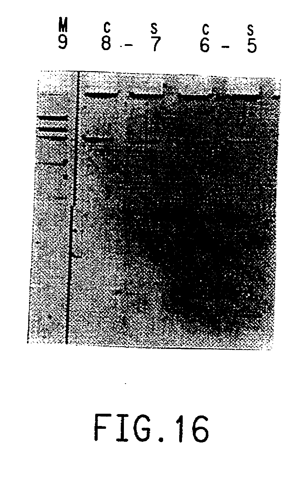

Figure 16 shows a test run on a sample of Italian sausage purchased at a local supermarket. Following the protocol above (24 h pre-enrichment per BAM; 1/10 grow back of original flask in (BHI) at 37 °C for 3 h), it was seen that *Salmonella* was present in the product as found in the store as evidenced by the typifying bands of the electrophoretic blots. The sample was evaluated in parallel by a commercial laboratory (Lancaster Laboratories, Inc., 2425 New Holland Pike, Lancaster, PA 17601). The reported result by standard BAM protocol was positive (Sample Number MBN 2266669).

Both procedures reveal the pathogen. The pre-enrichment time for both is 24 h, but the definitive assay of the instant invention takes under 8 h, including the 3 h, grow-back period. The BAM protocol (which requires selective enrichment, plating, etc.) takes an additional ninety-six hours for a negative finding and then an additional 18 h at a minimum to confirm a positive finding.

## Claims

1. A method of reliable detection of the presence of a specific organism in a complex mixture comprising:
i) providing a control reagent for polymerase chain reactions (PCR) comprising aliquots of:
a) an effective number of synthetic constructs of a characterizing DNA sequence structured with end subsequences that are complimentary to each other in reverse orientation;
b) an effective number of units of a single primer complimentary to the 3' end of each strand of said construct;
c) optionally an effective concentration of an intercalating dye;
d) optionally, a stabilizer;
e) an effective amount of a polymerase; and
f) an effective amount of deoxy-nucleotide;
ii) providing a test reagent comprising an effective number of units of a pair of primers, one member of the pair complimentary to one of the 3' ends of a target DNA, and the other member of the pair complimentary to the other 3' end of the target DNA, wherein one member of the pair is the same as the single primer of step i(b) above, and aliquots of c - f above;
iii) providing a non-selectively enriched test solution comprising lysed bacteria from a natural matrix suspected of containing a target organism;
iv) providing an effective solution of the solution of step iii with the reagent of step i in a single dose;
v) providing an effective solution of the solution of step iii with the reagent of step ii in a single dose;
vi) subjecting the solutions of steps iv and v to an effective number of PCR cycles;
vii) adding a tracking dye to the resultant solutions of step vi;
viii) subjecting the resultant solutions of step vii to electrophoretic separation (either PAGE or agarose gel) in parallel with a marker solution followed, optionally pre-stained with a nucleic acid-specific dye where said gel is not pre-stained with said nucleic acid-specific dye, by staining with a nucleic acid specific dye to produce identifying images; and
ix) optically analyzing the resultant images of step viii to definitively identify the presence or absence of the target organism.

2. The method of Claim 1 wherein said effective number of constructs is at least about 10.

3. The method of Claim 1 wherein the final concentration of said single primer is from about 0.01 micromolar to about 0.5 micromolar.

4. The method of Claim 1 wherein said optional intercalating dye is selected from the group consisting of nucleic acid dyes and asymeytric cyanine dyes and the effective concentration is from about 0.1 micromolar to about 6.0 micromolar.

5. The method of Claim 1 wherein said optional stabilizer is trehalose.

6. The method of Claim 1 wherein said synthetic sequence is modified recombinant pUC18 carrying Salmonella sequence with complimentary ends.

7. The method of Claim 1 wherein said control and test reagents are frozen into particles by means of a cryogenic liquid, said particles providing feedstock for tabletting.

8. The method of Claim 1 wherein said target bacterial sample was retrieved from the non-selectively enriched test solution of Step iii by incubation in the presence of a piece of Porex™ high density polyethylene whereby removal and processing said piece effectively processes said target bacterial sample.

9. The method of Claim 1 wherein said target bacterial sample was retrieved from the non-selectively enriched test solution of step,iii by a 1:10 dilution thereof followed by an effective grow-back incubation to provide an effective solution of the solution of step iii.

10. The method of claim 1 wherein said test reactant and said control reactant are tabletted.

11. The method of Claim 7 wherein the snow gun process is used for producing frozen particles of said control reactant, said particles providing the feedstock for tabletting.

12. A method of testing for a target bacterium using a control reactant for a polymerase chain reaction (PCR) comprising:
A) introducing into a control well a mixture of i) an effective amount of a control reactant comprising an effective number of copies of a single primer for a target DNA sequence, an effective number of copies of said target DNA sequence comprising a construct of an identifying sequence with end groups that are complimentary to each other in reverse orientation; ii) optionally an effective amount of compatible intercalating dye that does not adversely affect PCR amplification; iii) an effective amount of a polymerase; iv) an effective amount of a nucleotide; and, v) optionally, an effective amount of a stabilizer;
B) introducing into a test well a mixture of i) an effective amount of a test reactant comprising an effective amount of a pair of primers for a target sequence wherein one primer of the pair of primers is the same as the single primer of step A(i) above; ii) an effective amount of an intercalating dye that does not adversely affect PCR amplification; iii) an effective amount of a polymerase; iv) an effective amount of deoxynucleotides; and, v) optionally, an effective amount of a stabilizer;
C) adding to both the test well and the control well in a single dose addition an analysate comprising a liquid carrier including a buffer compatible with said polymerase, said buffer dosed with a sample from a source possibly containing a target bacterium and organic and/or inorganic extraneous matter, said analysate optionally subjected to a lysing step before said additional step, and said lysate optionally prepared by a dilution and regrowth procedure;
D) optionally mixing the contents in each of said wells;
E) lysing the bacterial cells of the analysate by subjecting both wells to a lysing temperature in those instances where in step C) the lysing was not done before said introduction;
F) heating the lysed contents of each well to effective pcr temperature and cycling for a selected number of cycles to provide a number of DNA copies sufficient for detection;
G) following step F), causing the dye in each well to fluoresce;
H) measuring light emitted from each of said wells; and
I) recording the target bacterium as present when adequate measurable light emission is observed in both wells or recording the target bacterium as absent when there is no adequate measurable emission in said test well and there is adequate emission in said control well or recording a faulty test when there is no adequate emission from said test well and said control well;
said adequacy of measurable light emission being determined by precalibration of light emission from the control reactant and comparison of the light emission from the control well and the test well after step H) with that calibration, deviations of light emission from the precalibrated value in the test well being ascribed to effects of extraneous matter as additive or subtractive, depending upon whether the observed value in said test well is above or below said calibrated value, permitting elimination of said effects.

13. The method of Claim 12 wherein said control reactant and said test reactant are tabletted.

14. The method of Claim 12 wherein said intercalating dye has a DNA partitioning coefficient in a ten percent ethanol/water solution substantially equal to or lower than 1 x 107.

15. The method of Claim 12 wherein said target bacterium was retrieved from a non-selective enrichment protocol by incubation of said bacterium with Porex™.

16. A method of testing for a target bacterium using a control reactant comprising:
A) adding to a control well i) an effective amount of a control reactant comprising an effective amount of a single primer for a target DNA sequence; ii) an effective number of copies of said target DNA sequence comprising a construct of an identifying sequence with end groups that are on the opposite strand; iii) an effective amount of a polymerase; iv) an effective amount of a nucleotide; and, v) optionally, an effective amount of a stabilizer;
B) adding to a test well i) an effective amount of a test reactant comprising an effective amount of a pair of primers specific to target DNA wherein one primer of the pair of primers is the same as the single primer of step A(i) above; ii) an effective amount of a polymerase; iii) an effective amount of a nucleotide; and, iv) optionally, an effective amount of a stabilizer;
C) adding to both the test well and the control well an analysate comprising a liquid carrier including a buffer compatible with said polymerase, said buffer dosed with a sample of a suspect target bacterium derived from a source possibly containing organic and/or inorganic extraneous matter, said analysate optionally subjected to a lysing step before said introduction;
D) optionally mixing the contents of each well;
E) lysing the bacterial content of the test well by subjecting both wells to a lysing temperature in those instance where in step C) the optional lysing was not done before said introduction;
F) heating the lysed contents of both wells to an effective pcr temperature and cycling for a selected number of cycles to provide a number of DNA replicates sufficient for detection;
G) adding an effective amount of an intercalating dye to both wells;
H) following step G), causing the dye in each well to fluoresce;
I) measuring any light emitted from each of said wells; and
J) identifying the target bacterium as present when adequate measurable emission is observed in both wells or identifying the target bacterium as absent when there is no adequate measurable emission in said test well and there is adequate emission in said control well or identifying the test as faulty when there is no adequate emission from said test well and said control well;
said adequacy being determined by precalibration of emission from the control reactant and comparison of the emission from the control well and the test well after step H) with that calibration, deviations of emission from the precalibrated value in the test well being ascribed to effects of extraneous matter and additive or subtractive, depending upon whether the observed value in said test well is above or below said calibrated value, to eliminate said effects.

17. The method of Claim 16 wherein said dosed sample of a suspect target bacterium is retrieved from a non-selective enrichment process by incubation in the presence of a piece of Porex™ high density polyethylene whereby removal and processing said piece effectively processes said target bacterial sample.

18. The method of Claim 16 wherein said dosed sample of a suspect target bacterium is retrieved from a non-selective enrichment process by a 1:10 dilution thereof followed by an effective grow-back incubation to provide an effective analysate.

19. The method of Claim 16 wherein said reactants are frozen by means of a cryogenic liquid into particles, said particles providing the feedstock for tabletting.

20. The method of Claim 19 wherein said test reactant and said control reactant are tabletted.

## Patentansprüche

1. Verfahren zum zuverlässigen Nachweis der Gegenwart eines bestimmten Organismus in einem komplexen Gemisch, mit den Schritten:
i) Bereitstellen eines Kontrollreagens für Polymerase-Kettenreaktionen (PCR), mit Aliquoten von:
a) einer wirksamen Anzahl synthetischer Konstrukte einer charakterisierenden DNA-Sequenz, die mit End-Subsequenzen strukturiert ist, welche in umgekehrter Orientierung komplementär zueinander sind;
b) einer wirksamen Anzahl von Einheiten eines einzelnen Starters, der zum 3'-Ende jedes Strangs des Konstrukts komplementär ist;
c) wahlweise einer wirksamen Konzentration eines interkalierenden Farbstoffs;
d) wahlweise einem Stabilisator;
e) einer wirksamen Menge einer Polymerase; und
f) einer wirksamen Menge eines Desoxynucleotids;
ii) Bereitstellen eines Testreagens, das aufweist: eine wirksame Anzahl von Einheiten eines Starterpaares, wobei ein Element des Paares komplementär zu einem der 3'-Enden einer Target-DNA und das andere Element des Paares komplementär zu dem anderen 3'-Ende der Target-DNA ist, wobei ein Element des Paares das gleiche wie der einzelne Starter im obigen Schritt i(b) ist, und Aliquoten gemäß den obigen Abschnitten c - f;
iii) Bereitstellen einer nichtselektiv angereicherten Testlösung, die lysierte Bakterien aus einer natürlichen Matrix aufweist, von der vermutet wird, daß sie einen Target-Organismus enthält;
iv) Bereitstellen einer wirksamen Lösung aus der Lösung von Schritt iii dem Reagens von Schritt i in einer einzigen Dosis;
v) Bereitstellen einer wirksamen Lösung aus der Lösung von Schritt iii und dem Reagens von Schritt ii in einer einzigen Dosis;
vi) Ausführen einer wirksamen Anzahl von PCR-Zyklen an den Lösungen der Schritte iv und v;
vii) Zugabe eines Tracer-Farbstoffs zu den resultierenden Lösungen von Schritt vi;
viii) Anwenden einer elektrophoretischen Trennung (entweder PAGE oder Agarosegel) auf die resultierenden Lösungen von Schritt vii parallel zum Verfolgen einer Markierungslösung, die wahlweise mit einem nucleinsäurespezifischen Farbstoff vorgefärbt wird, wobei das Gel nicht mit dem nucleinsäurespezifischen Farbstoff vorgefärbt wird, durch Einfärben mit einem nucleinsäurespezifischen Farbstoff zur Erzeugung von Identifikationsbildern; und
ix) optische Analyse der resultierenden Bilder von Schritt viii zur eindeutigen Feststellung der Gegenwart oder Abwesenheit des Target-Organismus.

2. Verfahren nach Anspruch 1, wobei die wirksame Anzahl von Konstrukten mindestens gleich 10 ist.

3. Verfahren nach Anspruch 1, wobei die Endkonzentration des einzelnen Starters etwa 0,01-mikromolar bis etwa 0,5-mikromolar ist.

4. Verfahren nach Anspruch 1, wobei der wahlfreie interkalierende Farbstoff aus der Gruppe ausgewählt ist, die aus Nucleinsäurefarbstoffen und asymmetrischen Cyaninfarbstoffen besteht, und wobei die wirksame Konzentration etwa 0,1-mikromolar bis etwa 6,0-mikromolar ist.

5. Verfahren nach Anspruch 1, wobei der wahlfreie Stabilisator Trehalose ist.

6. Verfahren nach Anspruch 1, wobei die synthetische Sequenz eine modifizierte rekombinante pUC18 ist, welche die Salmonella-Sequenz mit komplementären Enden trägt.

7. Verfahren nach Anspruch 1, wobei die Kontroll- und Restreagenzien durch ein Tieftemperaturfluid zu Teilchen gefroren sind, die das Einsatzmaterial zum Tablettieren bilden.

8. Verfahren nach Anspruch 1, wobei die Target-Bakterienprobe durch Inkubation in Gegenwart eines Stücks Porex™-Niederdruckpolyethylen aus der nichtselektiv angereicherten Testlösung von Schritt iii zurückgewonnen wurde, wodurch die Target-Bakterienprobe durch Entfernen und Verarbeiten des Stücks wirksam verarbeitet wird.

9. Verfahren nach Anspruch 1, wobei die Target-Bakterienprobe aus der nichtselektiv angereicherten Testlösung von Schritt iii durch eine 1:10-Verdünnung der Lösung mit anschließender Nachwachstums-Inkubation zurückgewonnen wurde, um eine wirksame Lösung aus der Lösung von Schritt iii bereitzustellen.

10. Verfahren nach Anspruch 1, wobei das Testreagens und das Kontrollreagens tablettiert sind.

11. Verfahren nach Anspruch 7, wobei zur Erzeugung gefrorener Teilchen des Kontrollreagens das Schneekanonenverfahren angewandt wird, wobei die Teilchen das Einsatzmaterial für das Tablettieren bilden.

12. Verfahren zum Prüfen eines Target-Bakteriums unter Verwendung eines Kontrollreagens für eine Polymerase-Kettenreaktion (PCR), mit den Schritten:
A) Einbringen eines Gemischs aus i) einer wirksamen Menge eines Kontrollreagens mit einer wirksamen Anzahl von Kopien eines einzelnen Starters für eine Target-DNA-Sequenz, einer wirksamen Anzahl von Kopien der Target-DNA-Sequenz, die ein Konstrukt aus einer Identifikationssequenz mit Endgruppen aufweisen, die in umgekehrter Orientierung komplementär zueinander sind; ii) wahlweise einer wirksamen Menge eines verträglichen interkalierenden Farbstoffs, der die PCR-Amplifikation nicht beeinträchtigt; iii) einer wirksamen Menge einer Polymerase; iv) einer wirksamen Menge eines Nucleotids; und v) wahlweise einer wirksamen Menge eines Stabilisators in eine Kontrollkavität;
B) Einbringen eines Gemischs aus i) einer wirksamen Menge eines Testreagens mit einer wirksamen Menge eines Starterpaares für eine Targetsequenz, wobei ein Starter des Starterpaares der gleiche ist wie der einzelne Starter im obigen Schritt A(i); ii) einer wirksamen Menge eines interkalierenden Farbstoffs, der die PCR-Amplifikation nicht beeinträchtigt; iii) einer wirksamen Menge einer Polymerase; iv) einer wirksamen Menge von Desoxynucleotiden; und v) wahlweise einer wirksamen Menge eines Stabilisators in eine Testkavität;
C) Zugabe eines Analysats mit einem flüssigen Träger, der einen mit der Polymerase verträglichen Puffer enthält, wobei dem Puffer eine Probe aus einer Quelle zudosiert ist, die möglicherweise ein Target-Bakterium und organische und/oder anorganische Fremdbestandteile enthält, wobei das Analysat wahlweise vor dem Zugabeschritt einem Lysierschritt ausgesetzt wird und das Lysat wahlweise durch ein Verdünnungs- und Nachwachsverfahren hergestellt wird, in einer einzigen Dosis sowohl in die Testkavität als auch in die Kontrollkavität;
D) wahlweise Vermischen der Inhalte in jeder der Kavitäten;
E) Lysieren der Bakterienzellen des Analysats durch Einwirkenlassen einer Lysiertemperatur auf beide Kavitäten in den Fällen, wo im Schritt C) das Lysieren nicht vor dem Einbringen ausgeführt wurde;
F) Erhitzen der lysierten Inhalte jeder Kavität auf eine wirksame PCR-Temperatur und Kreislaufführung über eine ausgewählte Anzahl von Zyklen, um eine für den Nachweis ausreichende Anzahl von DNA-Kopien bereitzustellen;
G) im Anschluß an Schritt F): Anregung des Farbstoffs in jeder Kavität zur Fluoreszenz;
H) Messen des von jeder der Kavitäten emittierten Lichts; und
I) Registrieren der Anwesenheit des Target-Bakteriums, wenn eine ausreichende meßbare Lichtemission in beiden Kavitäten beobachtet wird, oder Registrieren der Abwesenheit des Target-Bakteriums, wenn in der Testkavität keine ausreichende meßbare Emission und in der Kontrollkavität eine ausreichende Emission auftritt, oder Registrieren eines fehlerhaften Tests, wenn weder in der Testkavität noch in der Kontrollkavität eine ausreichende Emission auftritt;
wobei die ausreichende Stärke der meßbaren Lichtemission durch vorherige Eichung der Lichtemission vom Kontrollreagens und Vergleich der Lichtemission aus der Kontrollkavität und der Testkavität nach Schritt H) mit dieser Eichung festgelegt wird, wobei Abweichungen der Lichtemission vom vorgeeichten Wert im Test Effekten der Fremdbestandteile in additiver oder subtraktiver Form zugeschrieben werden, je nachdem, ob der beobachtete Wert in dem Test über oder unter dem Eichwert liegt, was die Elimination der Effekte gestattet.

13. Verfahren nach Anspruch 12, wobei das Kontrollreagens und das Testreagens tablettiert sind.

14. Verfahren nach Anspruch 12, wobei der interkalierende Farbstoff einen DNA-Verteilungskoeffizienten in einer zehnprozentigen Ethanol/Wasser-Lösung aufweist, der im wesentlichen kleiner oder gleich 1 x 10⁷ ist.

15. Verfahren nach Anspruch 12, wobei das Target-Bakterium aus einem nichtselektiven Anreicherungsprotokoll durch Inkubation des Bakteriums mit Porex™ zurückgewonnen wurde.

16. Testverfahren für ein Target-Bakterium unter Verwendung eines Kontrollreagens, mit den folgenden Schritten:
A) Zugabe i) einer wirksamen Menge eines Kontrollreagens mit einer wirksamen Menge eines einzelnen Starters für eine Target-DNA-Sequenz; ii) einer wirksamen Anzahl von Kopien der Target-DNA-Sequenz mit einem Konstrukt aus einer Identifikationssequenz mit Endgruppen, die sich auf dem gegenüberliegenden Strang befinden; iii) einer wirksamen Menge einer Polymerase; iv) einer wirksamen Menge eines Nucleotids; und v) wahlweise einer wirksamen Menge eines Stabilisators in eine Kontrollkavität;
B) Zugabe i) einer wirksamen Menge eines Testreagens mit einer wirksamen Menge eines für eine Target-DNA spezifischen Starterpaares, wobei ein Starter des Starterpaares der gleiche wie der einzelne Starter im obigen Schritt A(i) ist; ii) einer wirksamen Menge einer Polymerase; iii) einer wirksamen Menge eines Nucleotids; und iv) wahlweise einer wirksamen Menge eines Stabilisators in eine Testkavität; und
C) Zugabe eines Analysats mit einem flüssigen Träger, der einen mit der Polymerase verträglichen Puffer enthält, wobei dem Puffer eine Probe eines verdächtigen Target-Bakteriums zudosiert ist, die aus einer Quelle abgeleitet ist, die möglicherweise organische und/oder anorganische Fremdbestandteile enthält, wobei das Analysat wahlweise vor dem Einbringen einem Lysierschritt ausgesetzt wird, sowohl in die Testkavität als auch in die Kontrollkavität;
D) wahlweise Vermischen der Inhalte jeder Kavität;
E) Lysieren des bakteriellen Inhalts der Testkavität durch Einwirkenlassen einer Lysiertemperatur auf beide Kavitäten in den Fällen, wo im Schritt C) das wahlweise Lysieren nicht vor dem Einbringen ausgeführt wurde;
F) Erhitzen der lysierten Inhalte beider Kavitäten auf eine wirksame PCR-Temperatur und Kreislaufführung über eine ausgewählte Anzahl von Zyklen, um eine für den Nachweis ausreichende Anzahl von DNA-Replikaten herzustellen;
G) Zugabe einer wirksamen Menge eines interkalierenden Farbstoffs in beide Kavitäten;
H) im Anschluß an Schritt G): Anregung des Farbstoffs in jeder Kavität zur Fluoreszenz;
I) Messen einer etwaigen Lichtemission aus jeder der Kavitäten; und
J) Feststellen der Anwesenheit des Target-Bakteriums, wenn eine ausreichende meßbare Emission in beiden Kavitäten beobachtet wird, oder Feststellen der Abwesenheit des Target-Bakteriums, wenn in der Testkavität keine ausreichende meßbare Emission und in der Kontrollkavität eine ausreichende Emission auftritt, oder Feststellen eines fehlerhaften Tests, wenn weder in der Testkavität noch in der Kontrollkavität eine ausreichende Emission auftritt;
wobei die ausreichende Emissionsstärke durch vorherige Eichung der Emission vom Kontrollreagens und Vergleich der Emission aus der Kontrollkavität und der Testkavität nach dem Schritt H) mit dieser Eichung festgelegt wird, wobei Abweichungen der Emission vom vorgeeichten Wert im Test Effekten der Fremdbestandteile in additiver oder subtraktiver Form zugeschrieben werden, je nachdem, ob der beobachtete Wert in dem Test über oder unter dem Eichwert liegt, um die Effekte zu eliminieren.

17. Verfahren nach Anspruch 16, wobei die zudosierte Probe eines verdächtigen Target-Bakteriums aus einem nichtselektiven Anreicherungsprozeß durch Inkubation in Gegenwart eines Stücks Porex™-Niederdruckpolyethylen zurückgewonnen wird, wodurch die Target-Bakterienprobe durch Entfernen und Verarbeiten des Stücks wirksam verarbeitet wird.

18. Verfahren nach Anspruch 16, wobei die zudosierte Probe eines verdächtigen Target-Bakteriums aus einem nichtselektiven Anreicherungsprozeß durch 1:10-Verdünnung mit anschließender wirksamer Nachwachstums-Inkubation zurückgewonnen wird, um ein wirksames Analysat bereitzustellen.

19. Verfahren nach Anspruch 16, wobei die Reagenzien durch ein Tieftemperaturfluid zu Teilchen gefroren werden, wobei die Teilchen das Einsatzmaterial für das Tablettieren bilden.

20. Verfahren nach Anspruch 16, wobei das Testreagens und das Kontrollreagens tablettiert sind.

## Revendications

1. Un procédé de détection fiable de la présence d'un organisme spécifique dans un mélange complexe qui comprend :
i) l'utilisation d'un réactif témoin de réaction de chaîne polymérase (PCR) comprenant des aliquotes de :
a) un nombre efficace de constructions synthétiques d'une séquence d'ADN caractérisante structurée avec des sous-séquences terminales qui sont complémentaires l'une de l'autre dans des orientations inverses ;
b) un nombre efficace de motifs d'une amorce simple complémentaire de l'extrémité 3' de chaque brin de ladite construction ;
c) éventuellement une concentration efficace d'un colorant d'insertion ;
d) éventuellement, un stabilisant ;
e) une quantité efficace d'une polymérase ; et
f) une quantité efficace de désoxy-nucléotide ;
ii) l'utilisation d'un réactif d'essai comprenant un nombre efficace de motifs d'une paire d'amorces, l'un des éléments de ladite paire complémentaire de l'une des extrémités 3' d'un ADN cible et l'autre élément de la paire complémentaire de l'autre extrémité 3' de l'ADN cible, dans lequel un élément de la paire est le même que l'amorce simple de l'étape i (b) ci-dessus, et des aliquotes de c à f ci-dessus ;
iii) l'utilisation d'une solution d'essai non sélectivement enrichie comprend une bactérie lysée d'une matrice naturelle suspectée de renfermer l'organisme visé ;
iv) l'utilisation d'une solution efficace de la solution de l'étape iii avec le réactif de l'étape i en une dose unique ;
v) l'utilisation d'une solution efficace de la solution de l'étape iii avec le réactif de l'étape ii dans une dose unique ;
vi la soumission des solutions de l'étapes iv et v à un nombre efficace de cycles et de PRC ;
vii) l'addition d'un colorant traceur aux solutions résultantes de l'étape vi ;
viii) la soumission des solutions résultantes de l'étape vii à une séparation électrophorétique (soit PAGE soit sur gel d'agarose) en parallèle avec une solution de marqueur suivie, éventuellement, d'une précoloration à l'aide d'un colorant spécifique d'acide nucléique où ledit gel n'est pas précoloré à l'aide dudit colorant spécifique d'acide nucléique, par coloration avec le colorant spécifique d'acide nucléique pour obtenir une image d'identification et
ix) l'analyse optique des images résultant de l'étape viii pour définitivement identifier la présence ou l'absence de l'organisme visé.

2. Le procédé selon la revendication 1, dans lequel ledit nombre efficace de construction est d'au moins 10.

3. Le procédé selon la revendication 1, dans lequel la concentration finale de ladite amorce simple est de 0,01 micromolaire à environ 0,5 micromolaire.

4. Le procédé selon la revendication 1, dans lequel ledit colorant d'insertion éventuel est choisi dans le groupe consistant en colorant d'acide nucléique et colorant cyanine asymétrique et la concentration efficace est comprise entre environ 0.1 micromolaire à environ 6,0 micromolaires.

5. Le procédé selon la revendication 1, dans lequel ledit stabilisant éventuel est le tréhalose.

6. Le procédé selon la revendication 1, dans lequel ladite séquence synthétique est une séquence de Salmonelle portant du recombinant pUC18 avec des extrémités complémentaires.

7. Le procédé selon la revendication 1, dans lequel ledit témoin et lesdits réactifs d'essai sont congelés en particules à l'aide d'un liquide cryogénique. lesdites particules constituant une charge d'alimentation pour la mise en comprimés.

8. Le procédé selon la revendication 1, dans lequel ledit échantillon bactérien visé est récupéré d'une solution d'essai enrichie non-sélectivement dans l'étape iii par incubation en présence d'un morceau de polyéthylène haute densité Porex® selon lequel l'élimination et le traitement desdits morceaux permet de traiter effectivement lesdits échantillons bactériens visés.

9. Le procédé selon la revendication 1, dans lequel ledit échantillon bactérien visé est récupéré d'une solution d'essai enrichie non sélectivement de l'étape iii par une dilution de 1:10 suivie par une incubation de rétro-croissance pour obtenir une solution efficace de la solution de l'étape iii.

10. Le procédé selon la revendication 1, dans lequel ledit réactif d'essai et ledit réactif témoin sont sous forme de comprimés.

11. Le procédé selon la revendication 7, dans lequel on utilise le procédé du "canon à neige" pour obtenir des particules congelées desdits réactifs témoins, lesdites particules constituant une charge pour la mise sous forme de comprimé.

12. Un procédé de recherche d'une bactérie en utilisant un réactif témoin de réaction de chaîne polymérase PCR qui comprend :
A l'introduction dans un puits témoin, d'un mélange de i) une quantité efficace d'un réactif témoin comprenant un nombre efficace de copies d'une amorce simple pour une séquence d'ADN cible, un nombre efficace de copie desdites séquences d'ADN cible comprenant une construction d'une séquence identifiante avec des groupes terminaux qui sont complémentaires les uns des autres en orientation inverse ; ii) éventuellement une quantité efficace d'un colorant d'insertion compatible qui ne perturbe pas l'amplification du PCR; iii) une quantité efficace d'une polymérase ; iv) une quantité efficace d'un nucléotide ; et, v) éventuellement une quantité efficace d'un stabilisant ;
B) l'introduction dans un puits d'essai d'un mélange de mélange de i) et une quantité efficace d'un réactif d'essai comprenant une quantité efficace d'une paire d'amorces pour une séquence cible dans laquelle l'une des amorces de la paire d'amorces est la même que l'amorce simple de l'étape A (i) ci-dessus ; ii) une quantité efficace d'un colorant d'insertion qui ne perturbe pas à l'amplification de PCR; iii) une quantité efficace d'une polymérase ; iv) une quantité efficace de désoxy-nucléotide ; et, v) éventuellement une quantité efficace d'un stabilisant ;
C) l'addition à la fois au puits d'essai et au puits témoin, en une addition en une dose unique d'un analysat comprenant un support liquide comprenant un tampon compatible avec ladite polymérase, ledit tampon étant additionné d'une quantité dosée d'un échantillon provenant d'une source et contenant éventuellement le bactérium cible et une substance extranéenne organique et/ou inorganique, ledit analysat ayant été soumis éventuellement à une étape de lyse avant ladite étape d'addition, et ledit lysat étant éventuellement préparé par une méthode de dilution et de remise en culture ;
D) éventuellement le mélange du contenu de chacun desdits puits ;
E) la lyse les cellules bactériennes de l'analysat en soumettant lesdits puits à une température de lyse dans les cas où ou dans l'étape C), la lyse n'a pas été effectuée avant ladite introduction ;
F) le chauffage du contenu lysé de chacun des puits à une température de PCR efficace et on effectue un nombre de cycle choisis pour obtenir un nombre de copies de l'ADN suffisantes pour la détection ;
G) après l'étape F), on provoque la fluorescence du colorant dans chaque puits ;
H) on mesure la lumière émise de chacun desdits puits ; et
I) on enregistre que le bactérium visé est présent lorsqu'on observe une émission de lumière mesurable appropriée dans les deux puits ou on enregistre que le bactérium cible est absent lorsqu'il ne se produit pas d'émission mesurable adéquate dans lesdits puits d'essai et qu'il y a une émission adéquate dans ledit puits témoin, ou bien on enregistre que le test est erroné lorsqu'il n'y a pas d'émission adéquate ni dudit puits de mesure, ni dudit puits témoin ;
cette adéquation de l'émission de la lumière mesurable étant déterminée par un pré-étalonnage de l'émission lumineuse du réactif témoin et une comparaison de l'émission de lumière du puits témoin et du puits de mesure après l'étape H) avec cet étalonnage, les écarts d'émission de lumière de la valeur pré-étalonnée dans le test étant attribués à des effets de substance étrangère, comme additif ou substractif, selon que la valeur observée dans lesdits puits de mesure est supérieure ou inférieure à ladite valeur de référence, ce qui permet l'élimination desdits effets.

13. La méthode selon la revendication 12, dans laquelle ledit réactif témoin et ledit réactif de mesure sont sous forme de comprimés.

14. La méthode selon la revendication 12, dans laquelle ledit colorant intercalaire a un coefficient de partage d'ADN dans une solution aqueuse d'éthanol à 10 % sensiblement égal ou inférieur à 1 x 10⁷.

15. La méthode selon la revendication 12, dans laquelle ledit bactérium cible a été récupéré à partir d'un protocole d'enrichissement non sélectif par incubation dudit bactérium avec du Porex®.

16. Une méthode de test d'un bactérium cible, en utilisant un réactif témoin comprenant :
A) l'addition dans un puits témoin i) d'une quantité efficace d'un réactif témoin comprenant une quantité efficace d'une amorce simple de la séquence d'ADN cible ; ii) un nombre efficace de copies de ladite séquence d'ADN cible comprenant une construction d'une séquence d'identification avec des groupes terminaux qui sont sur le brin opposé ; iii) une quantité efficace de polymérase ; iv) une quantité efficace d'un nucléotide ; et v), éventuellement, une quantité efficace d'un stabilisant ;
B) addition dans le puits de mesure i) d'une quantité efficace du réactif d'épreuve comprenant une quantité efficace d'une paire d'amorces spécifiques de l'ADN cible dans laquelle l'une des amorces de la paire d'amorces est la même que l'amorce unique de l'étape A(i) ci-dessus, ii) une quantité efficace d'une polymérase ; iii) une quantité efficace d'un nucléotide et iv) éventuellement, une quantité efficace d'un stabilisant ;
C) addition à la fois au puits de mesure et au puits témoin d'un analysat comprenant un support liquide comprenant un tampon compatible avec ladite polymérase, ledit tampon dosé avec un échantillon de bactérium cible suspect dérivé d'une source contenant éventuellement du matériel extérieur organique et/ou inorganique, ledit analysat étant éventuellement soumis à une étape de lyse avant ladite introduction ;
D) le mélange éventuel du contenu de chaque puits ;
E) la lyse du contenu bactérien du puits de mesure, en soumettant les deux puits à une température de lyse dans le cas où, dans l'étape C, la lyse éventuelle n'a pas été effectuée avant ladite introduction ;
F) le chauffage du contenu lysé des deux puits à une température de PCR efficace et cyclage pendant un nombre de cycles choisis pour obtenir un nombre de répliques d'ADN suffisantes pour la détection ;
G) addition d'une quantité efficace d'un colorant intercalaire dans les deux puits ;
H) après l'étape G, excitation de la fluorescence du colorant dans chaque puits ;
I) mesure d'une lumière éventuellement émise par chacun desdits puits ; et
J) on considère que le bactérium cible est présent si on observe une émission mesurable adéquate dans les deux puits, ou on considère que le bactérium cible est absent quand on n'observe pas d'émission mesurable adéquate dans ledit puits de mesure et qu'il y a une émission adéquate dans ledit puits témoin, ou bien on considère que le test est erroné lorsqu'il n'y a pas d'émission adéquate, ni dans le puits témoin, ni dans le puits de mesure ;
ladite adéquation étant déterminée par un pré-étalonnage de l'émission du réactif témoin et comparaison de l'émission du puits témoin et du puits de mesure après l'étape H avec l'étalonnage, des écarts d'émission par rapport à la valeur de pré-étalonnage dans le puits de mesure étant considérés comme résultants de substances étrangères et étant additifs ou soustractifs selon que la valeur observée dans ledit puits de mesure est supérieure ou inférieure à la valeur d'étalonnage, pour éliminer lesdits effets.

17. La méthode selon la revendication 16, dans laquelle lesdits échantillons dosés d'un bactérium cible suspect est récupéré d'une opération d'enrichissement non sélective par incubation d'un morceau de polyéthylène haute densité Porex® et dans lequel le traitement dudit morceau traite efficacement ledit échantillon bactérien cible.

18. La méthode selon la revendication 16, dans laquelle ledit échantillon dosé d'un bactérium cible suspect est récupéré à partir d'une opération d'enrichissement non sélective par une dilution au 1:10 suivie d'une incubation efficace par rétro-culture pour obtenir un analysat efficace.

19. La méthode selon la revendication 16, dans laquelle lesdits réactifs sont congelés à l'aide d'un liquide cryogénique en particules, lesdites particules constituant la charge d'alimentation des comprimés.

20. La méthode selon la revendication 19, dans laquelle ledit réactif d'essai et ledit réactif témoin sont sous forme de comprimés.


### SEQUENCE LISTING

(1) GENERAL INFORMATION
   (i) APPLICANT:
      (A) NAME: E. I. DU PONT DE NEMOURS AND COMPANY
      (B) STREET: 1007 MARKET STREET
      (C) CITY: WILMINGTON
      (D) STATE: DELAWARE
      (E) COUNTRY: UNITED STATES OF AMERICA
      (F) POSTAL CODE (ZIP) : 19898
      (G) TELEPHONE: 302-892-8112
      (H) TELEFAX: 302-773-0164
      (I) TELEX: 6717325
   (ii) TITLE OF INVENTION: CONTROL FOR PCR
   (iii) NUMBER OF SEQUENCES: 1
   (iv) COMPUTER READABLE FORM
      (A) MEDIUM TYPE: FLOPPY DISK
      (B) COMPUTER: IBM COMPATIBLE
      (C) OPERATING SYSTEM: MS WINDOWS 3.1
      (D) SOFTWARE: WORD 2.0C
   (v) CURRENT APPLICATION DATA
      (A) APPLICATION NUMBER:
      (B) FILING DATE:
      (C) CLASSIFICATION:
   (vi) PRIOR APPLICATION DATA
      (A) APPLICATION NUMBER: 06/004,063
      (B) FILING DATE: SEPTEMBER 20, 1995
   (vii) ATTORNEY/AGENT INFORMATION:
      (A) NAME: FLOYD, LIN AXEMATHEY
      (B) REGISTRATION NUMBER: 33,692
      (C) REFERENCE/DOCKET NUMBER: MD-1066
(2) INFORMATION FOR SEQ ID NO:1:
   (i) SEQUENCE CHARACTERISTICS:
      (A) LENGTH: 779 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (xi) SEQUENCE DESCRIPTION: SEQ ID NO: 1:
   A GCCGGGACGC TTAATGCGGT TCTTTACCGC TTCCAGTGTG GCCTGAAAAC  51
   GCCACATGCC GACACCAGCG CCCGCCAGCG TGCCGAAACT GTAGAAACCA TGCATCATCG 111
   GCAGAACGGT TTTATTCAGC TCGCGTTCGA CCGCCGCGCC TTCGACATTA ATCGCCACTT 171
   CGGCGGCGCC AAAACTGGCG CCGAAAACGG CTAATCCAAG GGCAAAAATC AGCGGCGAGG 231
   CGCACCACAG CGCGACGCTA AGAATAACCA TCCCGGTTAC TGCACAGGTC ATCGTCGTGC 291
   GAATAACCTT CCGGGTGCCA AATCGTTTCA CCAGCCAGGC GGAACAAAGA ATACCGCTCA 351
   TTGAACCGAT AGAAAGCCCG AATAAGACCG CCCCCATTTC CGCGGTAGAG ACGGAAAGAA 411
   TATCCCGAAT AGCAGGCGTT CGGGTTGCCC AGGAGGCCAT CAGCAGTCCG GGTAAAAAGA 471
   AGAACATAAA CAGCGCCCAG GTACGGCGTT TTAAGGCGTT ACGTGAGGAG AGGACGGTCA 531
   TAGCGTCAGG CCAGAAAATA GAAGCGAGAG GTAAACATTA GCAAGCTTGT GTACATTTGT 591
   ACATATCATC GTCATACTTC ATTGTGCAGA CAGTTTTTAC TGTCTGTTTT TTCAGCGTAA 651
   GCGGCAGGCT ACTATCGCCT GCATCCTGAA TGAGATGTGG AACTCATCAT GAAAGAAAAT 711
   GCCGTAAGCG CGCCAATGAT CCTAAGCGAC GGGAAAAAAT AATTCAGGCC ACACTGGAAG 771
   CGGTAAAG                                                         779